# EUROPEAN PATENT SPECIFICATION

(11) **EP 0 914 613 B1**
(45) Date of publication and mention of the grant of the patent: **10.09.2003**
(21) Application number: 97921975.5
(22) Date of filing: 15.05.1997
(51) Int. Cl.: G01N 33/574, G01N 33/50, C12N 15/67, C07K 14/47

(54) **DP AND E2F PROTEIN NUCLEAR LOCALISATION SIGNALS AND THEIR USE**
NUKLEÄRE LOKALISIERUNGSSIGNALE VON DP UND E2F PROTEINE SOWIE DEREN VERWENDUNG
SIGNAUX DE LOCALISATION NUCLEAIRE DES PROTEINES DP ET E2F ET LEUR UTILISATION

(30) Priority: 15.05.1996 GB 9610195
(43) Date of publication of application: 12.05.1999
(73) Proprietor: Prolifix Limited, Abingdon, Oxfordshire OX14 4RY (GB)
(72) Inventor: LA THANGUE, Nicholas, B., University of Glasgow, Glasgow G12 8QQ (GB); DE LA LUNA, Susana, University of Glasgow, Glasgow G12 8QQ (GB)
(74) Representative: Brasnett, Adrian Hugh
(86) International application number: PCT/GB97/01324
(87) International publication number: WO 97/043647

(56) References cited:
- WO-A-93/15227
- WO-A-93/23539
- WO-A-94/10307
- WO-A-94/12521
- WO-A-96/01425
- WO-A-97/02354
- WU C.L. ET AL: "In vivo association of E2F and DP family proteins" MOL. CELL. BIOL. , vol. 15, 1995, pages 2536-2546, XP002041648 cited in the application
- ORMONDROYD E. ET AL: "A new member of the DP family, DP-3, ....." ONCOGENE, vol. 11, 1995, pages 1437-1446, XP002041649 cited in the application
- MAGAE J. ET AL: "Nuclear Localization of DP and E2F Transcription Factors......." JOURNAL OF CELL SCIENCE, vol. 109, August 1996, pages 1717-1726, XP002041650
- LAM E.W-F & LA THANGUE N.B.: "DP and E2F Proteins: Coordinating Transcription with Cell Cycle Progression." CURRENT OPINION IN CELL BIOLOGY, vol. 6, 1994, pages 859-866, XP002041651
- HELIN K, ET AL: "A cDNA Encoding a pRB-Binding Protein with properties of the Transcription Factor E2F" CELL, vol. 70, 1992, NA US, pages 337-350, XP002041808 cited in the application

### SEQUENCE LISTING

(1) GENERAL INFORMATION:
   (i) APPLICANT:
      (A) NAME: Medical Research Council
      (B) STREET: 20 Park Crescent
      (C) CITY: London
      (E) COUNTRY: United Kingdom
      (F) POSTAL CODE (ZIP): W1N 4AL

      (A) NAME: La Thangue, Nicholas Barrie
      (B) STREET: Institute of Biomedical and Life Sciences,
         Davidson Building, University of Glasgow
      (C) CITY: Glasgow
      (E) COUNTRY: United Kingdom
      (F) POSTAL CODE (ZIP): G12 8QQ

      (A) NAME: De La Luna, Susana
      (B) STREET: Institute of Biomedical and Life Sciences,
         Davidson Building, University of Glasgow
      (C) CITY: Glasgow
      (E) COUNTRY: United Kingdom
      (F) POSTAL CODE (ZIP): G12 8QQ
   (ii) TITLE OF INVENTION: DP and E2F protein nuclear localisation signals and their use
   (iii) NUMBER OF SEQUENCES: 21
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER: PCT/GB97/01324
   (vi) PRIOR APPLICATION DATA:
      (A) APPLICATION NUMBER: GB 9610195.1
      (B) FILING DATE: 15-MAY-1996
(2) INFORMATION FOR SEQ ID NO:1:
   (i) SEQUENCE CHARACTERISTICS:
      (A) LENGTH: 1385 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1338
   (xi) SEQUENCE DESCRIPTION: SEQ ID NO:1:
   ATG ACG GCA AAA AAT GTT GGT TTG CCA TCC ACA AAT GCA GAG CTG AGG        48
   Met Thr Ala Lys Asn Val Gly Leu Pro Ser Thr Asn Ala Glu Leu Arg
    1               5                  10                  15
   GGC TTT ATA GAT CAG AAT TTC AGT CCA ACG AAA GGT AAC ATT TCA CTT 96
   Gly Phe Ile Asp Gln Asn Phe Ser Pro Thr Lys Gly Asn Ile Ser Leu
   20 25 30
   GTT GCC TTT CCA GTT TCA AGC ACC AAC TCA CCA ACA AAG ATT TTA CCG 144
   Val Ala Phe Pro Val Ser Ser Thr Asn Ser Pro Thr Lys Ile Leu Pro
   35 40 45
   AAA ACC TTA GGG CCA ATA AAT GTG AAT GTT GGA CCC CAA ATG ATT ATA 192
   Lys Thr Leu Gly Pro Ile Asn Val Asn Val Gly Pro Gln Met Ile Ile
   50 55 60
   AGC ACA CCG CAG AGA ATT GCC AAT TCA GGA AGT GTT CTG ATT GGG AAT 240
   Ser Thr Pro Gln Arg Ile Ala Asn Ser Gly Ser Val Leu Ile Gly Asn
   65 70 75 80
   CCA TAT ACC CCT GCA CCC GCA ATG GTC ACT CAG ACT CAC ATA GCT GAG 288
   Pro Tyr Thr Pro Ala Pro Ala Met Val Thr Gln Thr His Ile Ala Glu
   85 90 95
   GCT GCT GGC TGG GTT CCC AGT GAT AGA AAA CGA GCT AGA GAA TTT ATA 336
   Ala Ala Gly Trp Val Pro Ser Asp Arg Lys Arg Ala Arg Glu Phe Ile
   100 105 110
   GAC TCT GAT TTT TCA GAA AGT AAA CGA AGC AAA AAA GGA GAT AAA AAT 384
   Asp Ser Asp Phe Ser Glu Ser Lys Arg Ser Lys Lys Gly Asp Lys Asn
   115 120 125
   GGG AAA GGC TTG AGA CAT TTT TCA ATG AAG GTG TGT GAG AAA GTT CAG 432
   Gly Lys Gly Leu Arg His Phe Ser Met Lys Val Cys Glu Lys Val Gln
   130 135 140
   CGG AAA GGC ACA ACT TCA TAC AAT GAG GTA GCT GAT GAG CTG GTA TCT 480
   Arg Lys Gly Thr Thr Ser Tyr Asn Glu Val Ala Asp Glu Leu Val Ser
   145 150 155 160
   GAG TTT ACC AAC TCA AAT AAC CAT CTG GCA GCT GAT TCG GCT TAT GAT 528
   Glu Phe Thr Asn Ser Asn Asn His Leu Ala Ala Asp Ser Ala Tyr Asp
   165 170 175
   CAG GAG AAC ATT AGA CGA AGA GTT TAT GAT GCT TTA AAT GTA CTA ATG 576
   Gln Glu Asn Ile Arg Arg Arg Val Tyr Asp Ala Leu Asn Val Leu Met
   180 185 190
   GCG ATG AAC ATA ATT TCA AAG GAA AAA AAA GAA ATC AAG TGG ATT GGC 624
   Ala Met Asn Ile Ile Ser Lys Glu Lys Lys Glu Ile Lys Trp Ile Gly
   195 200 205
   CTG CCT ACC AAT TCT GCT CAG GAA TGC CAG AAC CTG GAA ATC GAG AAG 672
   Leu Pro Thr Asn Ser Ala Gln Glu Cys Gln Asn Leu Glu Ile Glu Lys
   210 215 220
   CAG AGG CGG ATA GAA CGG ATA AAG CAG AAG CGA GCC CAG CTA CAA GAA 720
   Gln Arg Arg Ile Glu Arg Ile Lys Gln Lys Arg Ala Gln Leu Gln Glu
   225 230 235 240
   CTT CTC CTT CAG CAA ATT GCT TTT AAA AAC CTG GTA CAG AGA AAT CGA 768
   Leu Leu Leu Gln Gln Ile Ala Phe Lys Asn Leu Val Gln Arg Asn Arg
   245 250 255
   CAA AAT GAA CAA CAA AAC CAG GGC CCT CCA GCT GTG AAT TCC ACC ATT 816
   Gln Asn Glu Gln Gln Asn Gln Gly Pro Pro Ala Val Asn Ser Thr Ile
   260 265 270
   CAG CTG CCA TTT ATA ATC ATT AAT ACA AGC AGG AAA ACA GTC ATA GAC 864
   Gln Leu Pro Phe Ile Ile Ile Asn Thr Ser Arg Lys Thr Val Ile Asp
   275 280 285
   TGC AGC ATC TCC AGT GAC AAA TTT GAA TAC CTT TTT AAT TTT GAT AAC         912
   Cys Ser Ile Ser Ser Asp Lys Phe Glu Tyr Leu Phe Asn Phe Asp Asn
       290                 295                 300
   ACC TTT GAG ATC CAC GAC GAC ATA GAG GTA CTG AAG CGG ATG GGA ATG         960
   Thr Phe Glu Ile His Asp Asp Ile Glu Val Leu Lys Arg Met Gly Met
   305                 310                 315                 320
   TCC TTT GGT CTG GAG TCA GGC AAA TGC TCT CTG GAG GAT CTG AAA ATC        1008
   Ser Phe Gly Leu Glu Ser Gly Lys Cys Ser Leu Glu Asp Leu Lys Ile
                   325                 330                 335
   GCA AGA TCC CTG GTT CCA AAA GCT TTA GAA GGC TAT ATT ACA GAT ATC        1056
   Ala Arg Ser Leu Val Pro Lys Ala Leu Glu Gly Tyr Ile Thr Asp Ile
               340                 345                 350
   TCC ACA GGA CCT TCT TGG TTA AAT CAG GGA CTA CTT TTG AAC TCT ACC        1104
   Ser Thr Gly Pro Ser Trp Leu Asn Gln Gly Leu Leu Leu Asn Ser Thr
           355                 360                 365
   CAA TCA GTT TCA AAT TTA GAC CCG ACC ACC GGT GCC ACT GTA CCC CAA        1152
   Gln Ser Val Ser Asn Leu Asp Pro Thr Thr Gly Ala Thr Val Pro Gln
       370                 375                 380
   TCA AGT GTA AAC CAA GGG TTG TGC TTG GAT GCT GAA GTG GCC TTA GCA        1200
   Ser Ser Val Asn Gln Gly Leu Cys Leu Asp Ala Glu Val Ala Leu Ala
   385                 390                 395                 400
   ACT GGG CAG CTC CCT GCC TCA AAC AGT CAC CAG TCC AGC AGT GCA GCC        1248
   Thr Gly Gln Leu Pro Ala Ser Asn Ser His Gln Ser Ser Ser Ala Ala
                   405                 410                 415
   TCT CAC TTC TCG GAG TCC CGC GGC GAG ACC CCC TGT TCA TTC AAC GAT        1296
   Ser His Phe Ser Glu Ser Arg Gly Glu Thr Pro Cys Ser Phe Asn Asp
               420                 425                 430
   GAA GAT GAG GAA GAT GAA GAG GAG GAT CCC TCC TCC CCA GAA                1338
   Glu Asp Glu Glu Asp Glu Glu Glu Asp Pro Ser Ser Pro Glu
           435                 440                 445
   TAAAGACAGG AGAGAACTCA TGTTTTAAAA AAAAAAAAAA ACTCGAG                    1385
(2) INFORMATION FOR SEQ ID NO:2:
   (i) SEQUENCE CHARACTERISTICS:
      (A) LENGTH: 446 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Thr Ala Lys Asn Val Gly Leu Pro Ser Thr Asn Ala Glu Leu Arg
    1               5                  10                  15
   Gly Phe Ile Asp Gln Asn Phe Ser Pro Thr Lys Gly Asn Ile Ser Leu
               20                  25                  30
   Val Ala Phe Pro Val Ser Ser Thr Asn Ser Pro Thr Lys Ile Leu Pro
           35                  40                  45
   Lys Thr Leu Gly Pro Ile Asn Val Asn Val Gly Pro Gln Met Ile Ile
       50                  55                  60
   Ser Thr Pro Gln Arg Ile Ala Asn Ser Gly Ser Val Leu Ile Gly Asn
   65 70 75 80
   Pro Tyr Thr Pro Ala Pro Ala Met Val Thr Gln Thr His Ile Ala Glu
   85 90 95
   Ala Ala Gly Trp Val Pro Ser Asp Arg Lys Arg Ala Arg Glu Phe Ile
   100 105 110
   Asp Ser Asp Phe Ser Glu Ser Lys Arg Ser Lys Lys Gly Asp Lys Asn
   115 120 125
   Gly Lys Gly Leu Arg His Phe Ser Met Lys Val Cys Glu Lys Val Gln
   130 135 140
   Arg Lys Gly Thr Thr Ser Tyr Asn Glu Val Ala Asp Glu Leu Val Ser
   145 150 155 160
   Glu Phe Thr Asn Ser Asn Asn His Leu Ala Ala Asp Ser Ala Tyr Asp
   165 170 175
   Gln Glu Asn Ile Arg Arg Arg Val Tyr Asp Ala Leu Asn Val Leu Met
   180 185 190
   Ala Met Asn Ile Ile Ser Lys Glu Lys Lys Glu Ile Lys Trp Ile Gly
   195 200 205
   Leu Pro Thr Asn Ser Ala Gln Glu Cys Gln Asn Leu Glu Ile Glu Lys
   210 215 220
   Gln Arg Arg Ile Glu Arg Ile Lys Gln Lys Arg Ala Gln Leu Gln Glu
   225 230 235 240
   Leu Leu Leu Gln Gln Ile Ala Phe Lys Asn Leu Val Gln Arg Asn Arg
   245 250 255
   Gln Asn Glu Gln Gln Asn Gln Gly Pro Pro Ala Val Asn Ser Thr Ile
   260 265 270
   Gln Leu Pro Phe Ile Ile Ile Asn Thr Ser Arg Lys Thr Val Ile Asp
   275 280 285
   Cys Ser Ile Ser Ser Asp Lys Phe Glu Tyr Leu Phe Asn Phe Asp Asn
   290 295 300
   Thr Phe Glu Ile His Asp Asp Ile Glu Val Leu Lys Arg Met Gly Met
   305 310 315 320
   Ser Phe Gly Leu Glu Ser Gly Lys Cys Ser Leu Glu Asp Leu Lys Ile
   325 330 335
   Ala Arg Ser Leu Val Pro Lys Ala Leu Glu Gly Tyr Ile Thr Asp Ile
   340 345 350
   Ser Thr Gly Pro Ser Trp Leu Asn Gln Gly Leu Leu Leu Asn Ser Thr
   355 360 365
   Gln Ser Val Ser Asn Leu Asp Pro Thr Thr Gly Ala Thr Val Pro Gln
   370 375 380
   Ser Ser Val Asn Gln Gly Leu Cys Leu Asp Ala Glu Val Ala Leu Ala
   385 390 395 400
   Thr Gly Gln Leu Pro Ala Ser Asn Ser His Gln Ser Ser Ser Ala Ala
   405 410 415
   Ser His Phe Ser Glu Ser Arg Gly Glu Thr Pro Cys Ser Phe Asn Asp
               420                 425                 430
   Glu Asp Glu Glu Asp Glu Glu Glu Asp Pro Ser Ser Pro Glu
           435                 440                 445
(2) INFORMATION FOR SEQ ID NO:3:
   (i) SEQUENCE CHARACTERISTICS:
      (A) LENGTH: 1154 base pairs
      (B) TYPE: nucleic acid
      (C) STRANDEDNESS: single
      (D) TOPOLOGY: linear
   (ii) MOLECULE TYPES cDNA
   (ix) FEATURE:
      (A) NAME/KEY: CDS
      (B) LOCATION: 1..1107
   (xi) SEQUENCE DESCRIPTION: SEQ ID NO:3:
   ATG ATT ATA AGC ACA CCG CAG AGA ATT GCC AAT TCA GGA AGT GTT CTG        48
   Met Ile Ile Ser Thr Pro Gln Arg Ile Ala Asn Ser Gly Ser Val Leu
    1               5                  10                  15
   ATT GGG AAT CCA TAT ACC CCT GCA CCC GCA ATG GTC ACT CAG ACT CAC        96
   Ile Gly Asn Pro Tyr Thr Pro Ala Pro Ala Met Val Thr Gln Thr His
               20                  25                  30
   ATA GCT GAG GCT GCT GGC TGG GTT CCC AGT AAA CGA AGC AAA AAA GGA       144
   Ile Ala Glu Ala Ala Gly Trp Val Pro Ser Lys Arg Ser Lys Lys Gly
           35                  40                  45
   GAT AAA AAT GGG AAA GGC TTG AGA CAT TTT TCA ATG AAG GTG TGT GAG       192
   Asp Lys Asn Gly Lys Gly Leu Arg His Phe Ser Met Lys Val Cys Glu
       50                  55                  60
   AAA GTT CAG CGG AAA GGC ACA ACT TCA TAC AAT GAG GTA GCT GAT GAG       240
   Lys Val Gln Arg Lys Gly Thr Thr Ser Tyr Asn Glu Val Ala Asp Glu
   65                  70                  75                  80
   CTG GTA TCT GAG TTT ACC AAC TCA AAT AAC CAT CTG GCA GCT GAT TCG       288
   Leu Val Ser Glu Phe Thr Asn Ser Asn Asn His Leu Ala Ala Asp Ser
                   85                  90                  95
   GCT TAT GAT CAG GAG AAC ATT AGA CGA AGA GTT TAT GAT GCT TTA AAT       336
   Ala Tyr Asp Gln Glu Asn Ile Arg Arg Arg Val Tyr Asp Ala Leu Asn
               100                 105                 110
   GTA CTA ATG GCG ATG AAC ATA ATT TCA AAG GAA AAA AAA GAA ATC AAG       384
   Val Leu Met Ala Met Asn Ile Ile Ser Lys Glu Lys Lys Glu Ile Lys
           115                 120                 125
   TGG ATT GGC CTG CCT ACC AAT TCT GCT CAG GAA TGC CAG AAC CTG GAA       432
   Trp Ile Gly Leu Pro Thr Asn Ser Ala Gln Glu Cys Gln Asn Leu Glu
       130                 135                 140
   ATC GAG AAG CAG AGG CGG ATA GAA CGG ATA AAG CAG AAG CGA GCC CAG       480
   Ile Glu Lys Gln Arg Arg Ile Glu Arg Ile Lys Gln Lys Arg Ala Gln
   145                 150                 155                 160
   CTA CAA GAA CTT CTC CTT CAG CAA ATT GCT TTT AAA AAC CTG GTA CAG       528
   Leu Gln Glu Leu Leu Leu Gln Gln Ile Ala Phe Lys Asn Leu Val Gln
                   165                 170                 175
   AGA AAT CGA CAA AAT GAA CAA CAA AAC CAG GGC CCT CCA GCT GTG AAT        576
   Arg Asn Arg Gln Asn Glu Gln Gln Asn Gln Gly Pro Pro Ala Val Asn
               180                 185                 190
   TCC ACC ATT CAG CTG CCA TTT ATA ATC ATT AAT ACA AGC AGG AAA ACA        624
   Ser Thr Ile Gln Leu Pro Phe Ile Ile Ile Asn Thr Ser Arg Lys Thr
           195                 200                 205
   GTC ATA GAC TGC AGC ATC TCC AGT GAC AAA TTT GAA TAC CTT TTT AAT        672
   Val Ile Asp Cys Ser Ile Ser Ser Asp Lys Phe Glu Tyr Leu Phe Asn
       210                 215                 220
   TTT GAT AAC ACC TTT GAG ATC CAC GAC GAC ATA GAG GTA CTG AAG CGG        720
   Phe Asp Asn Thr Phe Glu Ile His Asp Asp Ile Glu Val Leu Lys Arg
   225                 230                 235                 240
   ATG GGA ATG TCC TTT GGT CTG GAG TCA GGC AAA TGC TCT CTG GAG GAT        768
   Met Gly Met Ser Phe Gly Leu Glu Ser Gly Lys Cys Ser Leu Glu Asp
                   245                 250                 255
   CTG AAA ATC GCA AGA TCC CTG GTT CCA AAA GCT TTA GAA GGC TAT ATT        816
   Leu Lys Ile Ala Arg Ser Leu Val Pro Lys Ala Leu Glu Gly Tyr Ile
               260                 265                 270
   ACA GAT ATC TCC ACA GGA CCT TCT TGG TTA AAT CAG GGA CTA CTT TTG        864
   Thr Asp Ile Ser Thr Gly Pro Ser Trp Leu Asn Gln Gly Leu Leu Leu
           275                 280                 285
   AAC TCT ACC CAA TCA GTT TCA AAT TTA GAC CCG ACC ACC GGT GCC ACT        912
   Asn Ser Thr Gln Ser Val Ser Asn Leu Asp Pro Thr Thr Gly Ala Thr
       290                 295                 300
   GTA CCC CAA TCA AGT GTA AAC CAA GGG TTG TGC TTG GAT GCT GAA GTG        960
   Val Pro Gln Ser Ser Val Asn Gln Gly Leu Cys Leu Asp Ala Glu Val
   305                 310                 315                 320
   GCC TTA GCA ACT GGG CAG CTC CCT GCC TCA AAC AGT CAC CAG TCC AGC       1008
   Ala Leu Ala Thr Gly Gln Leu Pro Ala Ser Asn Ser His Gln Ser Ser
                   325                 330                 335
   AGT GCA GCC TCT CAC TTC TCG GAG TCC CGC GGC GAG ACC CCC TGT TCA       1056
   Ser Ala Ala Ser His Phe Ser Glu Ser Arg Gly Glu Thr Pro Cys Ser
               340                 345                 350
   TTC AAC GAT GAA GAT GAG GAA GAT GAA GAG GAG GAT CCC TCC TCC CCA       1104
   Phe Asn Asp Glu Asp Glu Glu Asp Glu Glu Glu Asp Pro Ser Ser Pro
           355                 360                 365
   GAA TAAAGACAGG AGAGAACTCA TGTTTTAAAA AAAAAAAAAA ACTCGAG               1154
   Glu
(2) INFORMATION FOR SEQ ID NO:4:
   (i) SEQUENCE CHARACTERISTICS:
      (A) LENGTH: 369 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Met Ile Ile Ser Thr Pro Gln Arg Ile Ala Asn Ser Gly Ser Val Leu
   1 5 10 15
   Ile Gly Asn Pro Tyr Thr Pro Ala Pro Ala Met Val Thr Gln Thr His
               20                  25                  30
   Ile Ala Glu Ala Ala Gly Trp Val Pro Ser Lys Arg Ser Lys Lys Gly
           35                  40                  45
   Asp Lys Asn Gly Lys Gly Leu Arg His Phe Ser Met Lys Val Cys Glu
       50                  55                  60
   Lys Val Gln Arg Lys Gly Thr Thr Ser Tyr Asn Glu Val Ala Asp Glu
   65                  70                  75                  80
   Leu Val Ser Glu Phe Thr Asn Ser Asn Asn His Leu Ala Ala Asp Ser
                   85                  90                  95
   Ala Tyr Asp Gln Glu Asn Ile Arg Arg Arg Val Tyr Asp Ala Leu Asn
               100                 105                 110
   Val Leu Met Ala Met Asn Ile Ile Ser Lys Glu Lys Lys Glu Ile Lys
           115                 120                 125
   Trp Ile Gly Leu Pro Thr Asn Ser Ala Gln Glu Cys Gln Asn Leu Glu
       130                 135                 140
   Ile Glu Lys Gln Arg Arg Ile Glu Arg Ile Lys Gln Lys Arg Ala Gln
   145                 150                 155                 160
   Leu Gln Glu Leu Leu Leu Gln Gln Ile Ala Phe Lys Asn Leu Val Gln
                   165                 170                 175
   Arg Asn Arg Gln Asn Glu Gln Gln Asn Gln Gly Pro Pro Ala Val Asn
               180                 185                 190
   Ser Thr Ile Gln Leu Pro Phe Ile Ile Ile Asn Thr Ser Arg Lys Thr
           195                 200                 205
   Val Ile Asp Cys Ser Ile Ser Ser Asp Lys Phe Glu Tyr Leu Phe Asn
       210                 215                 220
   Phe Asp Asn Thr Phe Glu Ile His Asp Asp Ile Glu Val Leu Lys Arg
   225                 230                 235                 240
   Met Gly Met Ser Phe Gly Leu Glu Ser Gly Lys Cys Ser Leu Glu Asp
                   245                 250                 255
   Leu Lys Ile Ala Arg Ser Leu Val Pro Lys Ala Leu Glu Gly Tyr Ile
               260                 265                 270
   Thr Asp Ile Ser Thr Gly Pro Ser Trp Leu Asn Gln Gly Leu Leu Leu
           275                 280                 285
   Asn Ser Thr Gln Ser Val Ser Asn Leu Asp Pro Thr Thr Gly Ala Thr
       290                 295                 300
   Val Pro Gln Ser Ser Val Asn Gln Gly Leu Cys Leu Asp Ala Glu Val
   305                 310                 315                 320
   Ala Leu Ala Thr Gly Gln Leu Pro Ala Ser Asn Ser His Gln Ser Ser
                   325                 330                 335
   Ser Ala Ala Ser His Phe Ser Glu Ser Arg Gly Glu Thr Pro Cys Ser
               340                 345                 350
   Phe Asn Asp Glu Asp Glu Glu Asp Glu Glu Glu Asp Pro Ser Ser Pro
           355                 360                 365
   Glu
(2) INFORMATION FOR SEQ ID NO:5:
   (i) SEQUENCE CHARACTERISTICS:
      (A) LENGTH: 1157 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1110
   (xi) SEQUENCE DESCRIPTION: SEQ ID NO:5:
   ATG ATT ATA AGC ACA CCG CAG AGA ATT GCC AAT TCA GGA AGT GTT CTG     48
   Met Ile Ile Ser Thr Pro Gln Arg Ile Ala Asn Ser Gly Ser Val Leu
     1               5                  10                  15
   ATT GGG AAT CCA TAT ACC CCT GCA CCC GCA ATG GTC ACT CAG ACT CAC     96
   Ile Gly Asn Pro Tyr Thr Pro Ala Pro Ala Met Val Thr Gln Thr His
                20                  25                  30
   ATA GCT GAG GCT GCT GGC TGG GTT CCC AGT AAA CGA AGC AAA AAA GGA    144
   Ile Ala Glu Ala Ala Gly Trp Val Pro Ser Lys Arg Ser Lys Lys Gly
            35                  40                  45
   GAT AAA AAT GGG AAA GGC TTG AGA CAT TTT TCA ATG AAG GTG TGT GAG    192
   Asp Lys Asn Gly Lys Gly Leu Arg His Phe Ser Met Lys Val Cys Glu
        50                  55                  60
   AAA GTT CAG CGG AAA GGC ACA ACT TCA TAC AAT GAG GTA GCT GAT GAG    240
   Lys Val Gln Arg Lys Gly Thr Thr Ser Tyr Asn Glu Val Ala Asp Glu
    65                  70                  75                  80
   CTG GTA TCT GAG TTT ACC AAC TCA AAT AAC CAT CTG GCA GCT GAT TCG    288
   Leu Val Ser Glu Phe Thr Asn Ser Asn Asn His Leu Ala Ala Asp Ser
                    85                  90                  95
   CAG GCT TAT GAT CAG GAG AAC ATT AGA CGA AGA GTT TAT GAT GCT TTA    336
   Gln Ala Tyr Asp Gln Glu Asn Ile Arg Arg Arg Val Tyr Asp Ala Leu
               100                 105                 110
   AAT GTA CTA ATG GCG ATG AAC ATA ATT TCA AAG GAA AAA AAA GAA ATC    384
   Asn Val Leu Met Ala Met Asn Ile Ile Ser Lys Glu Lys Lys Glu Ile
           115                 120                 125
   AAG TGG ATT GGC CTG CCT ACC AAT TCT GCT CAG GAA TGC CAG AAC CTG    432
   Lys Trp Ile Gly Leu Pro Thr Asn Ser Ala Gln Glu Cys Gln Asn Leu
       130                 135                 140
   GAA ATC GAG AAG CAG AGG CGG ATA GAA CGG ATA AAG CAG AAG CGA GCC    480
   Glu Ile Glu Lys Gln Arg Arg Ile Glu Arg Ile Lys Gln Lys Arg Ala
   145                 150                 155                 160
   CAG CTA CAA GAA CTT CTC CTT CAG CAA ATT GCT TTT AAA AAC CTG GTA    528
   Gln Leu Gln Glu Leu Leu Leu Gln Gln Ile Ala Phe Lys Asn Leu Val
                   165                 170                 175
   CAG AGA AAT CGA CAA AAT GAA CAA CAA AAC CAG GGC CCT CCA GCT GTG    576
   Gln Arg Asn Arg Gln Asn Glu Gln Gln Asn Gln Gly Pro Pro Ala Val
               180                 185                 190
   AAT TCC ACC ATT CAG CTG CCA TTT ATA ATC ATT AAT ACA AGC AGG AAA        624
   Asn Ser Thr Ile Gln Leu Pro Phe Ile Ile Ile Asn Thr Ser Arg Lys
           195                 200                 205
   ACA GTC ATA GAC TGC AGC ATC TCC AGT GAC AAA TTT GAA TAC CTT TTT        672
   Thr Val Ile Asp Cys Ser Ile Ser Ser Asp Lys Phe Glu Tyr Leu Phe
       210                 215                 220
   AAT TTT GAT AAC ACC TTT GAG ATC CAC GAC GAC ATA GAG GTA CTG AAG        720
   Asn Phe Asp Asn Thr Phe Glu Ile His Asp Asp Ile Glu Val Leu Lys
   225                 230                 235                 240
   CGG ATG GGA ATG TCC TTT GGT CTG GAG TCA GGC AAA TGC TCT CTG GAG        768
   Arg Met Gly Met Ser Phe Gly Leu Glu Ser Gly Lys Cys Ser Leu Glu
                   245                 250                 255
   GAT CTG AAA ATC GCA AGA TCC CTG GTT CCA AAA GCT TTA GAA GGC TAT        816
   Asp Leu Lys Ile Ala Arg Ser Leu Val Pro Lys Ala Leu Glu Gly Tyr
               260                 265                 270
   ATT ACA GAT ATC TCC ACA GGA CCT TCT TGG TTA AAT CAG GGA CTA CTT        864
   Ile Thr Asp Ile Ser Thr Gly Pro Ser Trp Leu Asn Gln Gly Leu Leu
           275                 280                 285
   TTG AAC TCT ACC CAA TCA GTT TCA AAT TTA GAC CCG ACC ACC GGT GCC        912
   Leu Asn Ser Thr Gln Ser Val Ser Asn Leu Asp Pro Thr Thr Gly Ala
       290                 295                 300
   ACT GTA CCC CAA TCA AGT GTA AAC CAA GGG TTG TGC TTG GAT GCT GAA        960
   Thr Val Pro Gln Ser Ser Val Asn Gln Gly Leu Cys Leu Asp Ala Glu
   305                 310                 315                 320
   GTG GCC TTA GCA ACT GGG CAG CTC CCT GCC TCA AAC AGT CAC CAG TCC       1008
   Val Ala Leu Ala Thr Gly Gln Leu Pro Ala Ser Asn Ser His Gln Ser
                   325                 330                 335
   AGC AGT GCA GCC TCT CAC TTC TCG GAG TCC CGC GGC GAG ACC CCC TGT       1056
   Ser Ser Ala Ala Ser His Phe Ser Glu Ser Arg Gly Glu Thr Pro Cys
               340                 345                 350
   TCA TTC AAC GAT GAA GAT GAG GAA GAT GAA GAG GAG GAT CCC TCC TCC       1104
   Ser Phe Asn Asp Glu Asp Glu Glu Asp Glu Glu Glu Asp Pro Ser Ser
           355                 360                 365
   CCA GAA TAAAGACAGG AGAGAACTCA TGTTTTAAAA AAAAAAAAAA ACTCGAG           1157
   Pro Glu
       370
(2) INFORMATION FOR SEQ ID NO:6:
   (i) SEQUENCE CHARACTERISTICS:
      (A) LENGTH: 370 amino acids
      (B) TYPE: amino acid
      (D) TOPOLOGY: linear
   (ii) MOLECULE TYPE: protein
   (xi) SEQUENCE DESCRIPTION: SEQ ID NO:6:
   Met Ile Ile Ser Thr Pro Gln Arg Ile Ala Asn Ser Gly Ser Val Leu
    1               5                  10                  15
   Ile Gly Asn Pro Tyr Thr Pro Ala Pro Ala Met Val Thr Gln Thr His
               20                  25                  30
   Ile Ala Glu Ala Ala Gly Trp Val Pro Ser Lys Arg Ser Lys Lys Gly
           35                  40                  45
   Asp Lys Asn Gly Lys Gly Leu Arg His Phe Ser Met Lys Val Cys Glu
        50                  55                  60
   Lys Val Gln Arg Lys Gly Thr Thr Ser Tyr Asn Glu Val Ala Asp Glu
   65                  70                  75                  80
   Leu Val Ser Glu Phe Thr Asn Ser Asn Asn His Leu Ala Ala Asp Ser
                   85                  90                  95
   Gln Ala Tyr Asp Gln Glu Asn Ile Arg Arg Arg Val Tyr Asp Ala Leu
               100                 105                 110
   Asn Val Leu Met Ala Met Asn Ile Ile Ser Lys Glu Lys Lys Glu Ile
           115                 120                 125
   Lys Trp Ile Gly Leu Pro Thr Asn Ser Ala Gln Glu Cys Gln Asn Leu
       130                 135                 140
   Glu Ile Glu Lys Gln Arg Arg Ile Glu Arg Ile Lys Gln Lys Arg Ala
   145                 150                 155                 160
   Gln Leu Gln Glu Leu Leu Leu Gln Gln Ile Ala Phe Lys Asn Leu Val
                   165                 170                 175
   Gln Arg Asn Arg Gln Asn Glu Gln Gln Asn Gln Gly Pro Pro Ala Val
               180                 185                 190
   Asn Ser Thr Ile Gln Leu Pro Phe Ile Ile Ile Asn Thr Ser Arg Lys
           195                 200                 205
   Thr Val Ile Asp Cys Ser Ile Ser Ser Asp Lys Phe Glu Tyr Leu Phe
       210                 215                 220
   Asn Phe Asp Asn Thr Phe Glu Ile His Asp Asp Ile Glu Val Leu Lys
   225                 230                 235                 240
   Arg Met Gly Met Ser Phe Gly Leu Glu Ser Gly Lys Cys Ser Leu Glu
                   245                 250                 255
   Asp Leu Lys Ile Ala Arg Ser Leu Val Pro Lys Ala Leu Glu Gly Tyr
               260                 265                 270
   Ile Thr Asp Ile Ser Thr Gly Pro Ser Trp Leu Asn Gln Gly Leu Leu
           275                 280                 285
   Leu Asn Ser Thr Gln Ser Val Ser Asn Leu Asp Pro Thr Thr Gly Ala
       290                 295                 300
   Thr Val Pro Gln Ser Ser Val Asn Gln Gly Leu Cys Leu Asp Ala Glu
   305                 310                 315                 320
   Val Ala Leu Ala Thr Gly Gln Leu Pro Ala Ser Asn Ser His Gln Ser
                   325                 330                 335
   Ser Ser Ala Ala Ser His Phe Ser Glu Ser Arg Gly Glu Thr Pro Cys
               340                 345                 350
   Ser Phe Asn Asp Glu Asp Glu Glu Asp Glu Glu Glu Asp Pro Ser Ser
           355                 360                 365
   Pro Glu
       370
(2) INFORMATION FOR SEQ ID NO:7:
   (i) SEQUENCE CHARACTERISTICS:
      (A) LENGTH: 1202 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..1155
   (xi) SEQUENCE DESCRIPTION: SEQ ID NO:7:
   ATG ATT ATA AGC ACA CCG CAG AGA ATT GCC AAT TCA GGA AGT GTT CTG        48
   Met Ile Ile Ser Thr Pro Gln Arg Ile Ala Asn Ser Gly Ser Val Leu
    1               5                  10                  15
   ATT GGG AAT CCA TAT ACC CCT GCA CCC GCA ATG GTC ACT CAG ACT CAC        96
   Ile Gly Asn Pro Tyr Thr Pro Ala Pro Ala Met Val Thr Gln Thr His
               20                  25                  30
   ATA GCT GAG GCT GCT GGC TGG GTT CCC AGT GAT AGA AAA CGA GCT AGA       144
   Ile Ala Glu Ala Ala Gly Trp Val Pro Ser Asp Arg Lys Arg Ala Arg
           35                  40                  45
   GAA TTT ATA GAC TCT GAT TTT TCA GAA AGT AAA CGA AGC AAA AAA GGA       192
   Glu Phe Ile Asp Ser Asp Phe Ser Glu Ser Lys Arg Ser Lys Lys Gly
       50                  55                  60
   GAT AAA AAT GGG AAA GGC TTG AGA CAT TTT TCA ATG AAG GTG TGT GAG       240
   Asp Lys Asn Gly Lys Gly Leu Arg His Phe Ser Met Lys Val Cys Glu
   65                  70                  75                  80
   AAA GTT CAG CGG AAA GGC ACA ACT TCA TAC AAT GAG GTA GCT GAT GAG       288
   Lys Val Gln Arg Lys Gly Thr Thr Ser Tyr Asn Glu Val Ala Asp Glu
                   85                  90                  95
   CTG GTA TCT GAG TTT ACC AAC TCA AAT AAC CAT CTG GCA GCT GAT TCG       336
   Leu Val Ser Glu Phe Thr Asn Ser Asn Asn His Leu Ala Ala Asp Ser
               100                 105                 110
   GCT TAT GAT CAG GAG AAC ATT AGA CGA AGA GTT TAT GAT GCT TTA AAT       384
   Ala Tyr Asp Gln Glu Asn Ile Arg Arg Arg Val Tyr Asp Ala Leu Asn
           115                 120                 125
   GTA CTA ATG GCG ATG AAC ATA ATT TCA AAG GAA AAA AAA GAA ATC AAG       432
   Val Leu Met Ala Met Asn Ile Ile Ser Lys Glu Lys Lys Glu Ile Lys
       130                 135                 140
   TGG ATT GGC CTG CCT ACC AAT TCT GCT CAG GAA TGC CAG AAC CTG GAA       480
   Trp Ile Gly Leu Pro Thr Asn Ser Ala Gln Glu Cys Gln Asn Leu Glu
   145                 150                 155                 160
   ATC GAG AAG CAG AGG CGG ATA GAA CGG ATA AAG CAG AAG CGA GCC CAG       528
   Ile Glu Lys Gln Arg Arg Ile Glu Arg Ile Lys Gln Lys Arg Ala Gln
                   165                 170                 175
   CTA CAA GAA CTT CTC CTT CAG CAA ATT GCT TTT AAA AAC CTG GTA CAG       576
   Leu Gln Glu Leu Leu Leu Gln Gln Ile Ala Phe Lys Asn Leu Val Gln
               180                 185                 190
   AGA AAT CGA CAA AAT GAA CAA CAA AAC CAG GGC CCT CCA GCT GTG AAT        624
   Arg Asn Arg Gln Asn Glu Gln Gln Asn Gln Gly Pro Pro Ala Val Asn
           195                 200                 205
   TCC ACC ATT CAG CTG CCA TTT ATA ATC ATT AAT ACA AGC AGG AAA ACA        672
   Ser Thr Ile Gln Leu Pro Phe Ile Ile Ile Asn Thr Ser Arg Lys Thr
       210                 215                 220
   GTC ATA GAC TGC AGC ATC TCC AGT GAC AAA TTT GAA TAC CTT TTT AAT        720
   Val Ile Asp Cys Ser Ile Ser Ser Asp Lys Phe Glu Tyr Leu Phe Asn
   225                 230                 235                 240
   TTT GAT AAC ACC TTT GAG ATC CAC GAC GAC ATA GAG GTA CTG AAG CGG        768
   Phe Asp Asn Thr Phe Glu Ile His Asp Asp Ile Glu Val Leu Lys Arg
                   245                 250                 255
   ATG GGA ATG TCC TTT GGT CTG GAG TCA GGC AAA TGC TCT CTG GAG GAT        816
   Met Gly Met Ser Phe Gly Leu Glu Ser Gly Lys Cys Ser Leu Glu Asp
               260                 265                 270
   CTG AAA ATC GCA AGA TCC CTG GTT CCA AAA GCT TTA GAA GGC TAT ATT        864
   Leu Lys Ile Ala Arg Ser Leu Val Pro Lys Ala Leu Glu Gly Tyr Ile
           275                 280                 285
   ACA GAT ATC TCC ACA GGA CCT TCT TGG TTA AAT CAG GGA CTA CTT TTG        912
   Thr Asp Ile Ser Thr Gly Pro Ser Trp Leu Asn Gln Gly Leu Leu Leu
       290                 295                 300
   AAC TCT ACC CAA TCA GTT TCA AAT TTA GAC CCG ACC ACC GGT GCC ACT        960
   Asn Ser Thr Gln Ser Val Ser Asn Leu Asp Pro Thr Thr Gly Ala Thr
   305                 310                 315                 320
   GTA CCC CAA TCA AGT GTA AAC CAA GGG TTG TGC TTG GAT GCT GAA GTG       1008
   Val Pro Gln Ser Ser Val Asn Gln Gly Leu Cys Leu Asp Ala Glu Val
                   325                 330                 335
   GCC TTA GCA ACT GGG CAG CTC CCT GCC TCA AAC AGT CAC CAG TCC AGC       1056
   Ala Leu Ala Thr Gly Gln Leu Pro Ala Ser Asn Ser His Gln Ser Ser
               340                 345                 350
   AGT GCA GCC TCT CAC TTC TCG GAG TCC CGC GGC GAG ACC CCC TGT TCA       1104
   Ser Ala Ala Ser His Phe Ser Glu Ser Arg Gly Glu Thr Pro Cys Ser
           355                 360                 365
   TTC AAC GAT GAA GAT GAG GAA GAT GAA GAG GAG GAT CCC TCC TCC CCA       1152
   Phe Asn Asp Glu Asp Glu Glu Asp Glu Glu Glu Asp Pro Ser Ser Pro
       370                 375                 380
   GAA TAAAGACAGG AGAGAACTCA TGTTTTAAAA AAAAAAAAAA ACTCGAG               1202
   Glu
   385
(2) INFORMATION FOR SEQ ID NO:8:
   (i) SEQUENCE CHARACTERISTICS:
      (A) LENGTH: 385 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Ile Ile Ser Thr Pro Gln Arg Ile Ala Asn Ser Gly Ser Val Leu
   1               5                   10                  15
   Ile Gly Asn Pro Tyr Thr Pro Ala Pro Ala Met Val Thr Gln Thr His
   20 25 30
   Ile Ala Glu Ala Ala Gly Trp Val Pro Ser Asp Arg Lys Arg Ala Arg
   35 40 45
   Glu Phe Ile Asp Ser Asp Phe Ser Glu Ser Lys Arg Ser Lys Lys Gly
   50 55 60
   Asp Lys Asn Gly Lys Gly Leu Arg His Phe Ser Met Lys Val Cys Glu
   65 70 75 80
   Lys Val Gln Arg Lys Gly Thr Thr Ser Tyr Asn Glu Val Ala Asp Glu
   85 90 95
   Leu Val Ser Glu Phe Thr Asn Ser Asn Asn His Leu Ala Ala Asp Ser
   100 105 110
   Ala Tyr Asp Gln Glu Asn Ile Arg Arg Arg Val Tyr Asp Ala Leu Asn
   115 120 125
   Val Leu Met Ala Met Asn Ile Ile Ser Lys Glu Lys Lys Glu Ile Lys
   130 135 140
   Trp Ile Gly Leu Pro Thr Asn Ser Ala Gln Glu Cys Gln Asn Leu Glu
   145 150 155 160
   Ile Glu Lys Gln Arg Arg Ile Glu Arg Ile Lys Gln Lys Arg Ala Gln
   165 170 175
   Leu Gln Glu Leu Leu Leu Gln Gln Ile Ala Phe Lys Asn Leu Val Gln
   180 185 190
   Arg Asn Arg Gln Asn Glu Gln Gln Asn Gln Gly Pro Pro Ala Val Asn
   195 200 205
   Ser Thr Ile Gln Leu Pro Phe Ile Ile Ile Asn Thr Ser Arg Lys Thr
   210 215 220
   Val Ile Asp Cys Ser Ile Ser Ser Asp Lys Phe Glu Tyr Leu Phe Asn
   225 230 235 240
   Phe Asp Asn Thr Phe Glu Ile His Asp Asp Ile Glu Val Leu Lys Arg
   245 250 255
   Met Gly Met Ser Phe Gly Leu Glu Ser Gly Lys Cys Ser Leu Glu Asp
   260 265 270
   Leu Lys Ile Ala Arg Ser Leu Val Pro Lys Ala Leu Glu Gly Tyr Ile
   275 280 285
   Thr Asp Ile Ser Thr Gly Pro Ser Trp Leu Asn Gln Gly Leu Leu Leu
   290 295 300
   Asn Ser Thr Gln Ser Val Ser Asn Leu Asp Pro Thr Thr Gly Ala Thr
   305 310 315 320
   Val Pro Gln Ser Ser Val Asn Gln Gly Leu Cys Leu Asp Ala Glu Val
   325 330 335
   Ala Leu Ala Thr Gly Gln Leu Pro Ala Ser Asn Ser His Gln Ser Ser
   340 345 350
   Ser Ala Ala Ser His Phe Ser Glu Ser Arg Gly Glu Thr Pro Cys Ser
   355 360 365
   Phe Asn Asp Glu Asp Glu Glu Asp Glu Glu Glu Asp Pro Ser Ser Pro
   370 375 380
   Glu
   385
(2) INFORMATION FOR SEQ ID NO:9:
   (i) SEQUENCE CHARACTERISTICS:
      (A) LENGTH: 16 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:9:
   Ser Asp Arg Lys Arg Ala Arg Glu Phe Ile Asp Ser Asp Phe Ser Glu
   1 5 10 15
(2) INFORMATION FOR SEQ ID NO:10:
   (i) SEQUENCE CHARACTERISTICS:
      (A) LENGTH: 410 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO:10:
   Met Ala Lys Asp Ala Gly Leu Ile Glu Ala Asn Gly Glu Leu Lys Val
   1               5                   10                  15
   Phe Ile Asp Gln Asn Leu Ser Pro Gly Lys Gly Val Val Ser Leu Val
               20                  25                  30
   Ala Val His Pro Ser Thr Val Asn Pro Leu Gly Lys Gln Leu Leu Pro
           35                  40                  45
   Lys Thr Phe Gly Gln Ser Asn Val Asn Ile Ala Gln Gln Val Val Ile
       50                  55                  60
   Gly Thr Pro Gln Arg Pro Ala Ala Ser Asn Thr Leu Val Val Gly Ser
   65                  70                  75                  80
   Pro His Thr Pro Ser Thr His Phe Ala Ser Gln Asn Gln Pro Ser Asp
                   85                  90                  95
   Ser Ser Pro Trp Ser Ala Gly Lys Arg Asn Arg Lys Gly Glu Lys Asn
               100                 105                 110
   Gly Lys Gly Leu Arg His Phe Ser Met Lys Val Cys Glu Lys Val Gln
           115                 120                 125
   Arg Lys Gly Thr Thr Ser Tyr Asn Glu Val Ala Asp Glu Leu Val Ala
       130                 135                 140
   Glu Phe Ser Ala Ala Asp Asn His Ile Leu Pro Asn Glu Ser Ala Tyr
   145 150 155 160
   Asp Gln Lys Asn Ile Arg Arg Arg Val Tyr Asp Ala Leu Asn Val Leu
   165 170 175
   Met Ala Met Asn Ile Ile Ser Lys Glu Lys Lys Glu Ile Lys Trp Ile
   180 185 190
   Gly Leu Pro Thr Asn Ser Ala Gln Glu Cys Gln Asn Leu Glu Val Glu
   195 200 205
   Arg Gln Arg Arg Leu Glu Arg Ile Lys Gln Lys Gln Ser Gln Leu Gln
   210 215 220
   Glu Leu Ile Leu Gln Gln Ile Ala Phe Lys Asn Leu Val Gln Arg Asn
   225 230 235 240
   Arg His Ala Glu Gln Gln Ala Ser Arg Pro Pro Pro Pro Asn Ser Val
   245 250 255
   Ile His Leu Pro Phe Ile Ile Val Asn Thr Ser Lys Lys Thr Val Ile
   260 265 270
   Asp Cys Ser Ile Ser Asn Asp Lys Phe Glu Tyr Leu Phe Asn Phe Asp
   275 280 285
   Asn Thr Phe Glu Ile His Asp Asp Ile Glu Val Leu Lys Arg Met Gly
   290 295 300
   Met Ala Cys Gly Leu Glu Ser Gly Ser Cys Ser Ala Glu Asp Leu Lys
   305 310 315 320
   Met Ala Arg Ser Leu Val Pro Lys Ala Leu Glu Pro Tyr Val Thr Glu
   325 330 335
   Met Ala Gln Gly Thr Val Gly Gly Val Phe Ile Thr Thr Ala Gly Ser
   340 345 350
   Thr Ser Asn Gly Thr Arg Phe Ser Ala Ser Asp Leu Thr Asn Gly Ala
   355 360 365
   Asp Gly Met Leu Ala Thr Ser Ser Asn Gly Ser Gln Tyr Ser Gly Ser
   370 375 380
   Arg Val Glu Thr Pro Val Ser Tyr Val Gly Glu Asp Asp Glu Glu Asp
   385 390 395 400
   Asp Asp Phe Asn Glu Asn Asp Glu Asp Asp
   405 410
(2) INFORMATION FOR SEQ ID NO:11:
   (i) SEQUENCE CHARACTERISTICS:
      (A) LENGTH: 410 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO:11:
   Met Ala Lys Asp Ala Ser Leu Ile Glu Ala Asn Gly Glu Leu Lys Val
   1 5 10 15
   Phe Ile Asp Gln Asn Leu Ser Pro Gly Lys Gly Val Val Ser Leu Val
   20 25 30
   Ala Val His Pro Ser Thr Val Asn Thr Leu Gly Lys Gln Leu Leu Pro
   35 40 45
   Lys Thr Phe Gly Gln Ser Asn Val Asn Ile Thr Gln Gln Val Val Ile
   50 55 60
   Gly Thr Pro Gln Arg Pro Ala Ala Ser Asn Thr Ile Val Val Gly Ser
   65 70 75 80
   Pro His Thr Pro Asn Thr His Phe Val Ser Gln Asn Gln Thr Ser Asp
   85 90 95
   Ser Ser Pro Trp Ser Ala Gly Lys Arg Asn Arg Lys Gly Glu Lys Asn
   100 105 110
   Gly Lys Gly Leu Arg His Phe Ser Met Lys Val Cys Glu Lys Val Gln
   115 120 125
   Arg Lys Gly Thr Thr Ser Tyr Asn Glu Val Ala Asp Glu Leu Val Ala
   130 135 140
   Glu Phe Ser Ala Ala Asp Asn His Ile Leu Pro Asn Glu Ser Ala Tyr
   145 150 155 160
   Asp Gln Lys Asn Ile Arg Arg Arg Val Tyr Asp Ala Leu Asn Val Leu
   165 170 175
   Met Ala Met Asn Ile Ile Ser Lys Glu Lys Lys Glu Ile Lys Trp Ile
   180 185 190
   Gly Leu Pro Thr Asn Ser Ala Gln Glu Cys Gln Asn Leu Glu Val Glu
   195 200 205
   Arg Gln Arg Arg Leu Glu Arg Ile Lys Gln Lys Gln Ser Gln Leu Gln
   210 215 220
   Glu Leu Ile Leu Gln Gln Ile Ala Phe Lys Asn Leu Val Gln Arg Asn
   225 230 235 240
   Arg Gln Ala Glu Gln Gln Ala Arg Arg Pro Pro Pro Pro Asn Ser Val
   245 250 255
   Ile His Leu Pro Phe Ile Ile Val Asn Thr Ser Arg Lys Thr Val Ile
   260 265 270
   Asp Cys Ser Ile Ser Asn Asp Lys Phe Glu Tyr Leu Phe Asn Phe Asp
   275 280 285
   Asn Thr Phe Glu Ile His Asp Asp Ile Glu Val Leu Lys Arg Met Gly
   290 295 300
   Met Ala Cys Gly Leu Glu Ser Gly Asn Cys Ser Ala Glu Asp Leu Lys
   305 310 315 320
   Val Ala Arg Ser Leu Val Pro Lys Ala Leu Glu Pro Tyr Val Thr Glu
   325 330 335
   Met Ala Gln Gly Ser Ile Gly Gly Val Phe Val Thr Thr Thr Gly Ser
   340 345 350
   Thr Ser Asn Gly Thr Arg Leu Ser Ala Ser Asp Leu Ser Asn Gly Ala
   355 360 365
   Asp Gly Met Leu Ala Thr Ser Ser Asn Gly Ser Gln Tyr Ser Gly Ser
       370                 375                 380
   Arg Val Glu Thr Pro Val Ser Tyr Val Gly Glu Asp Asp Asp Asp Asp
   385                 390                 395                 400
   Asp Asp Phe Asn Glu Asn Asp Glu Glu Asp
                   405                 410
(2) INFORMATION FOR SEQ ID NO:12:
   (i) SEQUENCE CHARACTERISTICS:
      (A) LENGTH: 2457 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 87..1397
   (xi) SEQUENCE DESCRIPTION: SEQ ID NO:12:
   GGGATCGAGC CCTCGCCGAG GCCTGCCGCC ATGGGCCCGC GCCGCCGCCG CCGCCTGTCA        60
   CCCGGGCCGC GCGGGCCGTG AGCGTC ATG GCC TTG GCC GGG GCC CCT GCG GGC       113
                                Met Ala Leu Ala Gly Ala Pro Ala Gly
                                 1               5
   GGC CCA TGC GCG CCG GCG CTG GAG GCC CTG CTC GGG GCC GGC GCG CTG       161
   Gly Pro Cys Ala Pro Ala Leu Glu Ala Leu Leu Gly Ala Gly Ala Leu
    10                  15                  20                  25
   CGG CTG CTC GAC TCC TCG CAG ATC GTC ATC ATC TCC GCC GCG CAG GAC       209
   Arg Leu Leu Asp Ser Ser Gln Ile Val Ile Ile Ser Ala Ala Gln Asp
                    30                  35                  40
   GCC AGC GCC CCG CCG GCT CCC ACC GGC CCC GCG GCG CCC GCC GCC GGC       257
   Ala Ser Ala Pro Pro Ala Pro Thr Gly Pro Ala Ala Pro Ala Ala Gly
                45                  50                  55
   CCC TGC GAC CCT GAC CTG CTG CTC TTC GCC ACA CCG CAG GCG CCC CGG       305
   Pro Cys Asp Pro Asp Leu Leu Leu Phe Ala Thr Pro Gln Ala Pro Arg
            60                  65                  70
   CCC ACA CCC AGT GCG CCG CGG CCC GCG CTC GGC CGC CCG CCG GTG AAG       353
   Pro Thr Pro Ser Ala Pro Arg Pro Ala Leu Gly Arg Pro Pro Val Lys
        75                  80                  85
   CGG AGG CTG GAC CTG GAA ACT GAC CAT CAG TAC CTG GCC GAG AGC AGT       401
   Arg Arg Leu Asp Leu Glu Thr Asp His Gln Tyr Leu Ala Glu Ser Ser
    90                  95                 100                 105
   GGG CCA GCT CGG GGC AGA GGC CGC CAT CCA GGA AAA GGT GTG AAA TCC       449
   Gly Pro Ala Arg Gly Arg Gly Arg His Pro Gly Lys Gly Val Lys Ser
                   110                 115                 120
   CCG GGG GAG AAG TCA CGC TAT GAG ACC TCA CTG AAT CTG ACC ACC AAG       497
   Pro Gly Glu Lys Ser Arg Tyr Glu Thr Ser Leu Asn Leu Thr Thr Lys
               125                 130                 135
   CGC TTC CTG GAG CTG CTG AGC CAC TCG GCT GAC GGT GTC GTC GAC CTG       545
   Arg Phe Leu Glu Leu Leu Ser His Ser Ala Asp Gly Val Val Asp Leu
           140                 145                 150
   AAC TGG GCT GCC GAG GTG CTG AAG GTG CAG AAG CGG CGC ATC TAT GAC      593
   Asn Trp Ala Ala Glu Val Leu Lys Val Gln Lys Arg Arg Ile Tyr Asp
       155                 160                 165
   ATC ACC AAC GTC CTT GAG GGC ATC CAG CTC ATT GCC AAG AAG TCC AAG      641
   Ile Thr Asn Val Leu Glu Gly Ile Gln Leu Ile Ala Lys Lys Ser Lys
   170                 175                 180                 185
   AAC CAC ATC CAG TGG CTG GGC AGC CAC ACC ACA GTG GGC GTC GGC GGA      689
   Asn His Ile Gln Trp Leu Gly Ser His Thr Thr Val Gly Val Gly Gly
                   190                 195                 200
   CGG CTT GAG GGG TTG ACC CAG GAC CTC CGA CAG CTG CAG GAG AGC GAG      737
   Arg Leu Glu Gly Leu Thr Gln Asp Leu Arg Gln Leu Gln Glu Ser Glu
               205                 210                 215
   CAG CAG CTG GAC CAC CTG ATG AAT ATC TGT ACT ACG CAG CTG CGC CTG      785
   Gln Gln Leu Asp His Leu Met Asn Ile Cys Thr Thr Gln Leu Arg Leu
           220                 225                 230
   CTC TCC GAG GAC ACT GAC AGC CAG CGC CTG GCC TAC GTG ACG TGT CAG      833
   Leu Ser Glu Asp Thr Asp Ser Gln Arg Leu Ala Tyr Val Thr Cys Gln
       235                 240                 245
   GAC CTT CGT AGC ATT GCA GAC CCT GCA GAG CAG ATG GTT ATG GTG ATC      881
   Asp Leu Arg Ser Ile Ala Asp Pro Ala Glu Gln Met Val Met Val Ile
   250                 255                 260                 265
   AAA GCC CCT CCT GAG ACC CAG CTC CAA GCC GTG GAC TCT TCG GAG AAC      929
   Lys Ala Pro Pro Glu Thr Gln Leu Gln Ala Val Asp Ser Ser Glu Asn
                   270                 275                 280
   TTT CAG ATC TCC CTT AAG AGC AAA CAA GGC CCG ATC GAT GTT TTC CTG      977
   Phe Gln Ile Ser Leu Lys Ser Lys Gln Gly Pro Ile Asp Val Phe Leu
               285                 290                 295
   TGC CCT GAG GAG ACC GTA GGT GGG ATC AGC CCT GGG AAG ACC CCA TCC     1025
   Cys Pro Glu Glu Thr Val Gly Gly Ile Ser Pro Gly Lys Thr Pro Ser
           300                 305                 310
   CAG GAG GTC ACT TCT GAG GAG GAG AAC AGG GCC ACT GAC TCT GCC ACC     1073
   Gln Glu Val Thr Ser Glu Glu Glu Asn Arg Ala Thr Asp Ser Ala Thr
       315                 320                 325
   ATA GTG TCA CCA CCA CCA TCA TCT CCC CCC TCA TCC CTC ACC ACA GAT     1121
   Ile Val Ser Pro Pro Pro Ser Ser Pro Pro Ser Ser Leu Thr Thr Asp
   330                 335                 340                 345
   CCC AGC CAG TCT CTA CTC AGC CTG GAG CAA GAA CCG CTG TTG TCC CGG     1169
   Pro Ser Gln Ser Leu Leu Ser Leu Glu Gln Glu Pro Leu Leu Ser Arg
                   350                 355                 360
   ATG GGC AGC CTG CGG GCT CCC GTG GAC GAG GAC CGC CTG TCC CCG CTG     1217
   Met Gly Ser Leu Arg Ala Pro Val Asp Glu Asp Arg Leu Ser Pro Leu
               365                 370                 375
   GTG GCG GCC GAC TCG CTC CTG GAG CAT GTG CGG GAG GAC TTC TCC GGC     1265
   Val Ala Ala Asp Ser Leu Leu Glu His Val Arg Glu Asp Phe Ser Gly
           380                 385                 390
   CTC CTC CCT GAG GAG TTC ATC AGC CTT TCC CCA CCC CAC GAG GCC CTC     1313
   Leu Leu Pro Glu Glu Phe Ile Ser Leu Ser Pro Pro His Glu Ala Leu
       395                 400                 405
   GAC TAC CAC TTC GGC CTC GAG GAG GGC GAG GGC ATC AGA GAC CTC TTC     1361
   Asp Tyr His Phe Gly Leu Glu Glu Gly Glu Gly Ile Arg Asp Leu Phe
   410                 415                 420                 425
   GAC TGT GAC TTT GGG GAC CTC ACC CCC CTG GAT TTC TGACAGGGCT          1407
   Asp Cys Asp Phe Gly Asp Leu Thr Pro Leu Asp Phe
                   430                 435
   TGGAGGGACC AGGGTTTCCA GAGTAGCTCA CCTTGTCTCT GCAGCCCTGG AGCCCCCTGT    1467
   CCCTGGCCGT CCTCCCAGCC TGTTTGGAAA CATTTAATTT ATACCCCTCT CCTCTGTCTC    1527
   CAGAAGCTTC TAGCTCTGGG GTCTGGCTAC CGCTAGGAGG CTGAGCAAGC CAGGAAGGGA    1587
   AGGAGTCTGT GTGGTGTGTA TGTGCATGCA GCCTACACCC ACACGTGTGT ACCGGGGGTG    1647
   AATGTGTGTG AGCATGTGTG TGTGCATGTA CCGGGGAATG AAGGTGAACA TACACCTCTG    1707
   TGTGTGCACT GCAGACACGC CCCAGTGTGT CCACATGTGT GTGCATGAGT CCATCTCTGC    1767
   GCGTGGGGGG GCTCTAACTG CACTTTCGGC CCTTTTGCTC GTGGGGTCCC ACAAGGCCCA    1827
   GGGCAGTGCC TGCTCCCAGA ATCTGGTGCT CTGACCAGGC CAGGTGGGGA GCCTTTGGCT    1887
   GGCTGGGCGT GTAGGACGGT GAGAGCACTT CTGTCTTAAA GGTTTTTTCT GATTGAAGCT    1947
   TTAATGGAGC GTTATTTATT TATCGAGGCC TCTTTGGTGA GCCTGGGGAA TCAGCAAAAG    2007
   GGGAGGAGGG GTGTGGGGTT GATACCCCAA CTCCCTCTAC CCTTGAGCAA GGGCAGGGGT    2067
   CCCTGAGCTG TTCTTCTGCC CCATACTGAA GGAACTGAGG CCTGGGTGAT TTATTTATTG    2127
   GGAAAGTGAG GGAGGGAGAC AGACTGACTG ACAGCCATGG GTGGTCAGAT GGTGGGGTGG    2187
   GCCCTCTCCA GGGGGCCAGT TCAGGGCCCA GCTGCCCCCC AGGATGGATA TGAGATGGGA    2247
   GAGGTGAGTG GGGGACCTTC ACTGATGTGG GCAGGAGGGG TGGTGAAGGC CTCCCCCAGC    2307
   CCAGACCCTG TGGTCCCTCC TGCAGTGTCT GAAGCGCCTG CCTCCCCACT GCTCTGCCCC    2367
   ACCCTCCAAT CTGCACTTTG ATTTGCTTCC TAACAGCTCT GTTCCCTCCT GCTTTGGTTT    2427
   TAATAAATAT TTTGATGACG TTAAAAAAAA                                     2457
(2) INFORMATION FOR SEQ ID NO:13:
   (i) SEQUENCE CHARACTERISTICS:
      (A) LENGTH: 437 amino acids
      (B) TYPE: amino acid
      (D) TOPOLOGY: linear
   (ii) MOLECULE TYPE: protein
   (xi) SEQUENCE DESCRIPTION: SEQ ID NO:13:
   Met Ala Leu Ala Gly Ala Pro Ala Gly Gly Pro Cys Ala Pro Ala Leu
   1               5                   10                  15
   Glu Ala Leu Leu Gly Ala Gly Ala Leu Arg Leu Leu Asp Ser Ser Gln
               20                  25                  30
   Ile Val Ile Ile Ser Ala Ala Gln Asp Ala Ser Ala Pro Pro Ala Pro
           35                  40                  45
   Thr Gly Pro Ala Ala Pro Ala Ala Gly Pro Cys Asp Pro Asp Leu Leu
       50                  55                  60
   Leu Phe Ala Thr Pro Gln Ala Pro Arg Pro Thr Pro Ser Ala Pro Arg
   65                  70                  75                  80
   Pro Ala Leu Gly Arg Pro Pro Val Lys Arg Arg Leu Asp Leu Glu Thr
                   85                  90                  95
   Asp His Gln Tyr Leu Ala Glu Ser Ser Gly Pro Ala Arg Gly Arg Gly
               100                 105                 110
   Arg His Pro Gly Lys Gly Val Lys Ser Pro Gly Glu Lys Ser Arg Tyr
           115                 120                 125
   Glu Thr Ser Leu Asn Leu Thr Thr Lys Arg Phe Leu Glu Leu Leu Ser
       130                 135                 140
   His Ser Ala Asp Gly Val Val Asp Leu Asn Trp Ala Ala Glu Val Leu
   145                 150                 155                 160
   Lys Val Gln Lys Arg Arg Ile Tyr Asp Ile Thr Asn Val Leu Glu Gly
                   165                 170                 175
   Ile Gln Leu Ile Ala Lys Lys Ser Lys Asn His Ile Gln Trp Leu Gly
               180                 185                 190
   Ser His Thr Thr Val Gly Val Gly Gly Arg Leu Glu Gly Leu Thr Gln
           195                 200                 205
   Asp Leu Arg Gln Leu Gln Glu Ser Glu Gln Gln Leu Asp His Leu Met
       210                 215                 220
   Asn Ile Cys Thr Thr Gln Leu Arg Leu Leu Ser Glu Asp Thr Asp Ser
   225                 230                 235                 240
   Gln Arg Leu Ala Tyr Val Thr Cys Gln Asp Leu Arg Ser Ile Ala Asp
                   245                 250                 255
   Pro Ala Glu Gln Met Val Met Val Ile Lys Ala Pro Pro Glu Thr Gln
               260                 265                 270
   Leu Gln Ala Val Asp Ser Ser Glu Asn Phe Gln Ile Ser Leu Lys Ser
           275                 280                 285
   Lys Gln Gly Pro Ile Asp Val Phe Leu Cys Pro Glu Glu Thr Val Gly
       290                 295                 300
   Gly Ile Ser Pro Gly Lys Thr Pro Ser Gln Glu Val Thr Ser Glu Glu
   305                 310                 315                 320
   Glu Asn Arg Ala Thr Asp Ser Ala Thr Ile Val Ser Pro Pro Pro Ser
                   325                 330                 335
   Ser Pro Pro Ser Ser Leu Thr Thr Asp Pro Ser Gln Ser Leu Leu Ser
               340                 345                 350
   Leu Glu Gln Glu Pro Leu Leu Ser Arg Met Gly Ser Leu Arg Ala Pro
           355                 360                 365
   Val Asp Glu Asp Arg Leu Ser Pro Leu Val Ala Ala Asp Ser Leu Leu
       370                 375                 380
   Glu His Val Arg Glu Asp Phe Ser Gly Leu Leu Pro Glu Glu Phe Ile
   385                 390                 395                 400
   Ser Leu Ser Pro Pro His Glu Ala Leu Asp Tyr His Phe Gly Leu Glu
                   405                 410                 415
   Glu Gly Glu Gly Ile Arg Asp Leu Phe Asp Cys Asp Phe Gly Asp Leu
               420                 425                 430
   Thr Pro Leu Asp Phe
   435
(2) INFORMATION FOR SEQ ID NO:14:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PRIMER"
   (xi) SEQUENCE DESCRIPTION: SEQ ID NO:14:
   GCTCTAGAGC CCAGTATAGA 20
(2) INFORMATION FOR SEQ ID NO:15:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PRIMER"
   (xi) SEQUENCE DESCRIPTION: SEQ ID NO:15:
   GCTCTAGATG TCTCAAGCCT TTCCC 25
(2) INFORMATION FOR SEQ ID NO:16:
   (i) SEQUENCE CHARACTERISTICS:
      (A) LENGTH: 13 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:16:
   Asp Glu Glu Asp Glu Glu Glu Asp Pro Ser Ser Pro Glu
   1 5 10
(2) INFORMATION FOR SEQ ID NO:17:
   (i) SEQUENCE CHARACTERISTICS:
      (A) LENGTH: 15 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:17:
   Val Ala Leu Ala Thr Gly Gln Leu Pro Ala Ser Asn Ser His Gln
   1               5                   10                  15
(2) INFORMATION FOR SEQ ID NO:18:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PRIMER"
   (xi) SEQUENCE DESCRIPTION: SEQ ID NO:18:
   CACCCGCAAT GGTCACT                                                17
(2) INFORMATION FOR SEQ ID NO:19:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PRIMER"
   (xi) SEQUENCE DESCRIPTION: SEQ ID NO:19:
   ATGTCTCAAG CCTTTCCC                                               18
(2) INFORMATION FOR SEQ ID NO:20:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PRIMER"
   (xi) SEQUENCE DESCRIPTION: SEQ ID NO:20:
   GATAGAAAAC GAGCTAGAG 19
(2) INFORMATION FOR SEQ ID NO:21:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "PRIMER"
   (xi) SEQUENCE DESCRIPTION: SEQ ID NO:21:
   TTCTGAGAAA TCAGAGTCTA 20

## Description

The present invention relates to the use of the E region of the transcription factor DP-3 as a target for novel assays and its use as a nuclear localisation signal.

The orderly progress of cells through the cell cycle involves a number of control points which assess the status of the intracellular and extracellular environment. A major control point, occurring as cells enter S phase, involves the cellular transcription factor E2F, a molecule implicated in the regulation of S phase gene expression (Nevins, 1992; La Thangue, 1994; Müller, 1995; Weinberg, 1995). An important for E2F in early cell cycle control is suggested by the nature of the proteins which influence its transcriptional activity. For example, members of the group of pocket proteins, exemplified by the retinoblastoma tumour suppressor gene product (pRb), repress the transcriptional activity of E2F (Hiebert *et al*., 1992; Zamanian and La Thangue, 1992; 1993; Schwarz *et al*., 1993; Wolf *et al.,* 1995). The ability to repress E2F correlates with the capacity of pRb, or its relatives, to negatively regulate early cell cycle progression (Hiebert *et al.,* 1992; Zamanian and La Thangue, 1992; Hinds *et al.,* 1992; Zhu *et al.,* 1993; 1995a). Furthermore, growth arrest caused by high level expression of pRb can be overcome by increasing the level of E2F (Zhu *et al*., 1993), implying that E2F is a principal physiological target through which pRb exerts its effects on the cell cycle. Another group of molecules which regulate cell cycle transitions, the cyclins and their associated catalytic regulatory subunits, also interact with and control the activity of E2F (Bandara *et al.,* 1991; Lees *et al*., 1992; Zhu *et al*., 1995b). Cyclins A, E and D together with an appropriate catalytic subunit can influence the biological activity of pocket proteins (Hinds *et al.,* 1992; Dowdy *et al.,* 1993; Ewen *et al.*, 1993; Sherr, 1993), and direct phosphorylation by cyclinA-cdk2 is believed to interfere with the DNA binding activity of E2F (Krek *et al.,* 1994; 1995).

The physiological regulation of E2F activity imparted by these afferent signalling proteins can be subverted by viral oncoproteins, such as adenovirus E1a, human papilloma virus E7 and SV40 large T antigen, through their ability to release active E2F by sequestering pocket proteins and cyclin/cdk complexes (Bandara and La Thangue, 1991; Chellappan *et al.,* 1991; 1992; Morris *et al.,* 1993). This property correlates with the ability of these viral oncoproteins to transform tissue culture cells, again implicating E2F as an important physiological target in virally-medicated oncogenesis.

Considerable progress has been made in elucidating the composition of E2F. It is now known the E2F DNA binding activity defined in mammalian cell extracts is a generic activity caused by an array of DNA binding heterodimers made up from two distinct families of proteins, known as E2F and DP (La Thangue, 1994). Five members of the E2F family, from E2F-1 to E2F-5, have been isolated, each protein possessing preferential specificity for pocket proteins (Helin *et al*., 1992; Kaelin *et al.,* 1992; Shan *et al* 1992; Ivey-Hoyle *et al.,* 1993; Lees *et al*., 1993; Beijersbergen *et al*., 1994; Ginsberg *et al.,* 1994; Buck *et al.,* 1995; Hijmans *et al.,* 1995; Sardet *et al.,* 1995). For example, E2F-1 is regulated by pRb, and E2F-4 by p107 and p130 (Helin *et al.,* 1993a; Flemington *et al.,* 1993; Beijersbergen *et al*., 1994; 1995; Ginsberg *et al*., 1994; Vairo *et al*., 1995). Three members of the DP family are known (Girling *et al*., 11993; 1994; Ormondroyd *et al.,* 1995; Wu *et al*., 1995; Zhang and Chellappan, 1995), DP-1 being a widespread and constitutive component of physiological E2F during cell cycle progression in some cell types (Girling *et al.,* 1993; Bandara *et al*., 1994). Supporting their role as dominant regulators of the cell cycle, both E2F and DP proteins have been shown to possess proto-oncogenic activity (Johnson *et al.,* 1994; Jooss *et al.,* 1995).

Our previous characterisation of DP-3 indicated that it is a novel member of the DP family of proteins and that its RNA undergoes extensive alternative splicing (Ormondroyd *et al*., 1995). Processing events in the 5' untranslated region and coding sequence of the RNA give rise to a range of products present in both cell lines and tissues (Ormondroyd *et al.,* 1995). A sequence of 16 amino acid residues within the N-terminal region of the DNA binding domain, known as the E region, is one such region subject to the alternative splicing of DP-3 RNA. Further, in the four DP-3 protein products which have been characterised, α and δ constitute E+ forms, whereas β and γ are E- variants (Ormondroyd *et al*., 1995). Although E-; extensive sequence conservation is apparent across the DP protein family, a comparison of the known DP protein sequences indicated that they fall into two categories, being either E+ or for example, DP-1 is an E-variant.

### Description of the Drawing.

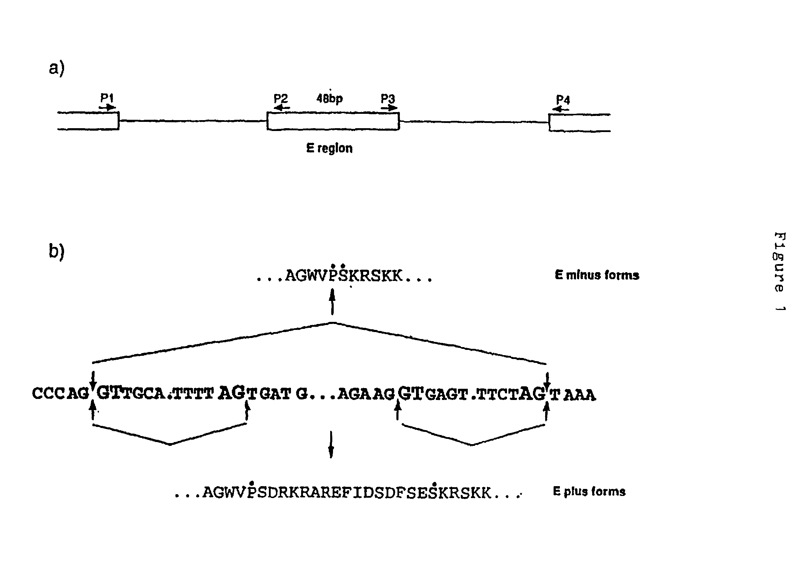

Figure 1 shows the DP-3 E-region exon and the patterns of alternate splicing which give rise to E+ and E- forms of DP-3.

### Disclosure of the Invention.

In the present study, we have defined a role for the E region by showing that its inclusion contributes to an alternatively spliced nuclear localization signal: specifically, E+ DP-3 proteins accumulate in the nuclei whereas E- proteins, including DP-1, fail to do so. Without the E region, DP proteins rely upon an alternative mechanism which involves an interaction with an appropriate E2F family member, for example E2F-1, for nuclear accumulation. These data define two mechanisms of control in the nuclear accumulation of E2F transcription factor influenced by alternative splicing of a nuclear localization signal and subunit composition, and indicate a hitherto unexpected and novel level of control in regulating the levels of the nuclear E2F/DP heterodimer.

The present invention thus provides an assay for a putative regulator of cell cycle progression which comprises:
a) expressing in a cell a protein comprising
   (i) the E region and sufficient C-terminal residues thereof of a DP-3 protein to provide a functional nuclear localisation signal (NLS), said E region having a sequence: or a variant thereof modified by substitution, insertion or deletion wherein said variant retains NLS activity and wherein modification by substitution is substitution of from 1 to 4 amino acids and modification by insertion or deletion is insertion or deletion of from 1 to 3 amino acids; and
   (ii) a marker for nuclear localization fused to (i); and
b) determining the degree of nuclear localization of said protein in the presence and absence of a putative regulator of cell cycle progression in order to determine whether or not said putative regulator is a regulator of the cell cycle.

In a further embodiment of the invention, the finding that DP proteins such as DP-1 lack an NLS indicate that the complex of such DP proteins with an E2F (such as E2F-1) are localised in the nucleus by the presence of an NLS on the E2F protein. The DP-3 NLS is not homologous to the E2F NLS. Thus the E2F NLS forms a further target for antagonists of nuclear localisation of the DP/E2F complex, particularly complexes such as DP-1/E2F-1 which do not comprise an E region. We have identified the nuclear localisation signal region in E2F-1. This region is identified as residues 85-91 of the human E2F-1 sequence shown as SEQ ID NO. 12 below. Thus the invention also provides an assay for a putative regulator of cell cycle progression which comprises:
a) expressing in a cell a protein comprising
   (i) the nuclear localisation signal (NLS) of E2F-1, said NLS having a sequence comprising residues 85-91 of SEQ ID NO:12, or a variant thereof modified by substitution insertion or deletion wherein said variant retains NLS activity and wherein modification by substitution is substitution of from 1 to 4 amino acids and modification by insertion or deletion is insertion or deletion of from 1 to 3 amino acids; and
   (ii) a marker for nuclear localization, and
b) determining the degree of nuclear localization of said protein in the presence and absence of a putative regulator of cell cycle progression in order to determine whether or not said putative regulator is a regulator of the cell cycle.

The proteins defined in parts "a" above will be referred to as the "a protein comprising an NLS-region" and the like for the sake of brevity.

In one embodiment, the E region comprises the sequence:

However, this E region is derived from the murine DP-3 gene and other E regions may be used, for example the human E region or other mammalian E regions. The murine DP-3 alpha, beta, gamma and delta genes are shown as SEQ ID NOs. 1 and 2, 3 and 4, 5 and 6, and 7 and 8 respectively. Other DP-3 genes may be obtained by routine cloning methods. For example, the human DP-3 gene may be cloned by probing a cDNA or genomic library with a nucleic acid probe derived from either a known human DP-gene (e.g. DP-1) and/or the murine DP-3 gene, and positive clones selected and sequenced for the human DP-3 gene. Similar techniques may be used for other mammalian DP-3 genes and will be readily apparent to those of skill in the art.

As described herein, the E region requires a number of C-terminal residues found in the DP-3 sequence in order to function as.an NLS. Desirably, from 6 to 50, e.g 8 to 30 and preferably from 8 to 20 C-terminal residues are used.

Similarly, the NLS of E2F-1 may be used with accompanying Nor C-terminal residues from the natural sequence of this protein, although these are not essential for the activity of the NLS.

Although assays of the invention are preferably based upon naturally occurring NLS-regions sequences and associated C-terminal regions thereof sufficient to act as an NLS, these sequences may also be modified by substitution, deletion or insertion provided that the function of these sequences is substantially retained. The retention of function may be tested for in accordance with the description and examples herein. Such modified and functional NLS-regions are included within the definition of the terms "an E region of a DP-3 protein" and "the nuclear localisation signal of E2F-1".

For example, from 1 to 4 substitutions may be made and these are preferably conservative substitutions. Examples of conservative substitutions include those set out in the following table, where amino acids on the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |
| OTHER | | N Q D E |

Where deletions or insertions are made, these are preferably limited in number for example from 1 to 3 of each.

The cell in which the assay may be conducted is any suitable eukaryotic cell in which the NLS-regions function as nuclear localisation signals. Suitable cell types include yeast, insect or mammalian cells, e.g. primate cells such as COS7 cells.

In the assay according to the invention the marker may be any polypeptide sequence which allows detection of the presence and location (i.e. cytoplasmic vs nuclear) of the protein comprising an NLS region. Suitable markers include an antigenic determinant bindable by an antibody, an enzyme capable of causing a colour change to a substrate or a luciferase enzyme.

In a preferred embodiment, the marker comprises a transcription factor or subunit thereof, which transcription factor is capable of activating an indicator gene. This embodiment avoids the need for detailed examination of the cell to determine where the marker has located. In this embodiment the activation of transcription of the indicator gene will show that the NLS-regions have been located the protein in the nucleus.

For example, in a preferred embodiment of the invention the protein may comprise a heterologous DNA binding domain such as that of the yeast transcription factor GAL 4. The GAL 4 transcription factor comprises two functional domains.
These domains are the DNA binding domain (DBD) and the transcriptional activation domain (TAD). By fusing an NLS-region to one of those domains and expressing the other domain in the cell, a functional GAL 4 transcription factor is restored only when two proteins enter the nucleus and interact. Thus, interaction of the proteins may be measured by the use of an indicator gene linked to a GAL 4 DNA binding site which is capable of activating transcription of said reporter gene. This assay format is described by Fields and Song, 1989, Nature 340; 245-246. Other transcriptional activator domains may be used in place of the GAL4 TAD, for example the viral VP16 activation domain (Fields and Jang, 1990). In general, fusion proteins comprising DNA binding domains and/or activation domains may be made.

The indicator gene may comprise, for example, chloramphenicol acetyl transferase (CAT) or a luciferase.

The NLS may be located at the C-terminal or N-terminal of the marker gene. The NLS may be within all or part of the DP-3 or E2F protein from which it originates, or may be solely the NLS sequences identified above which provide the necessary NLS function. Thus fragments of DP-3 or an E2F (e.g. E2F-1) of from 15 to 400, eg from 20 to 100 or from 30 to 50 amino acids comprising the NLS may be used. Where the NLS is fused to the N- or C-terminus of a marker gene, the fusion may comprise further sequences at its N- or C-terminus where this is desired or necessary.

In any format, the assay may be used to screen peptides which regulate the function of an NLS. Regulation of the function includes antagonising the function to prevent nuclear localisation although regulators may also be agonists which enhance localisation. Regulation of the NLS may lead to effects such as enhanced cell division, blocking of cell cycle progression or apoptosis, the latter two being particularly preferred. Candidate regulators identified in accordance with the invention may be tested on cells with wild-type DP and E2F proteins to confirm the effect of regulating the NLS.

Such regulators will be useful either in themselves as potential regulators of cell proliferation or as models for rational drug design, e.g. by modelling the tertiary structure of the antagonist and devising chemical analogues which mimic the structure.

Candidate regulators include peptides comprising all or part of a sequence which is from 60 to 100% homologous (identical) to a portion of an NLS region of the same length. Extracts of plants which contain several characterised or uncharacterised components may also be used.

Antibodies directed to the NLS regions form a further class of putative regulator compounds. Candidate regulator antibodies may be characterised and their binding regions determined to provide single chain antibodies and fragments thereof which are responsible for regulating the interaction.

Other candidate regulator compounds may be based on modelling the 3-dimensional structure of the NLS regions and using rational drug design to provide potential inhibitor compounds with particular molecular shape, size and charge characteristics.

An regulator substance identified using the present invention may be peptide or non-peptide in nature. Non-peptide "small molecules" are often preferred for many *in vivo* pharmaceutical uses. Accordingly, a mimetic or mimick of the substance (particularly if a peptide) may be designed for pharmaceutical use.

The designing of mimetics to a known pharmaceutically active compound is a known approach to the development of pharmaceuticals based on a "lead" compound. This might be desirable where the active compound is difficult or expensive to synthesise or where it is unsuitable for a particular method of administration, e.g. peptides are not well suited as active agents for oral compositions as they tend to be quickly degraded by proteases in the alimentary canal. Mimetic design, synthesis and testing may be used to avoid randomly screening large number of molecules for a target property.

There are several steps commonly taken in the design of a mimetic from a compound having a given target property. Firstly, the particular parts of the compound that are critical and/or important in determining the target property are determined. In the case of a peptide, this can be done by systematically varying the amino acid residues in the peptide, e.g. by substituting each residue in turn. These parts or residues constituting the active region of the compound are known as its "pharmacophore".

Once the pharmacophore has been found, its structure is modelled to according its physical properties, e.g. stereochemistry, bonding, size and/or charge, using data from a range of sources, e.g. spectroscopic techniques, X-ray diffraction data and NMR. Computational analysis, similarity mapping (which models the charge and/or volume of a pharmacophore, rather than the bonding between atoms) and other techniques can be used in this modelling process.

In a variant of this approach, the three-dimensional structure of the ligand and its binding partner are modelled. This can be especially useful where the ligand and/or binding partner change conformation on binding, allowing the model to take account of this the design of the mimetic.

A template molecule is then selected onto which chemical groups which mimic the pharmacophore can be grafted. The template molecule and the chemical groups grafted on to it can conveniently be selected so that the mimetic is easy to synthesise, is likely to be pharmacologically acceptable, and does not degrade *in vivo*, while retaining the biological activity of the lead compound. The mimetic or mimetics found by this approach can then be screened to see whether they have the target property, or to what extent they exhibit it. Further optimisation or modification can then be carried out to arrive at one or more final mimetics for *in vivo* or clinical testing.

Antibodies may be obtained using techniques which are standard in the art. Methods of producing antibodies include immunising a mammal (e.g. mouse, rat, rabbit, horse, goat, sheep or monkey) with the protein or a fragment thereof. Antibodies may be obtained from immunised animals using any of a variety.of techniques known in the art, and screened, preferably using binding of antibody to antigen of interest. For instance, Western blotting techniques or immunoprecipitation may be used (Armitage *et al*., 1992, Nature 357: 80-82). Isolation of antibodies and/or antibody-producing cells from an animal may be accompanied by a step of sacrificing the animal.

As an alternative or supplement to immunising a mammal with a peptide, an antibody specific for a protein may be obtained from a recombinantly produced library of expressed immunoglobulin variable domains, e.g. using lambda bacteriophage or filamentous bacteriophage which display functional immunoglobulin binding domains on their surfaces; for instance see WO92/01047. The library may be naive, that is constructed from sequences obtained from an organism which has not been immunised with any of the proteins (or fragments), or may be one constructed using sequences obtained from an organism which has been exposed to the antigen of interest.

Antibodies according to the present invention may be modified in a number of ways. Indeed the term "antibody" should be construed as covering any binding substance having a binding domain with the required specificity. Thus the invention covers antibody fragments, derivatives, functional equivalents and homologues of antibodies, including synthetic molecules and molecules whose shape mimicks that of an antibody enabling it to bind an antigen or epitope.

Examples of antibody fragments, capable of binding an antigen or other binding partner are the Fab fragment consisting of the VL, VH, Cl and CH1 domains; the Fd fragment consisting of the VH and CH1 domains; the Fv fragment consisting of the VL and VH domains of a single arm of an antibody; the dAb fragment which consists of a VH domain; isolated CDR regions and F(ab')2 fragments, a bivalent fragment including two Fab fragments linked by a disulphide bridge at the hinge region. Single chain Fv fragments are also included.

A hybridoma producing a monoclonal antibody according to the present invention may be subject to genetic mutation or other changes. It will further be understood by those skilled in the art that a monoclonal antibody can be subjected to the techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP184187A, GB 2188638A or EP-A-0239400. Cloning and expression of chimeric antibodies are described in EP-A-0120694 and EP-A-0125023.

The amount of a putative regulator which may be screened in the assay of the invention desirably will be selected to be a concentration which is within 100 fold (above or below) the amount of an NLS-region-containing protein in the cell. By way of guidance this will mean that typically, from about 0.01 to 100 nM concentrations of putative regulator compound may be used, for example from 0.1 to 10 nM.

The assay of the invention may be conducted using transient expression vectors or stably transfected cells. In either case, the protein comprising an NLS-region will be encoded by nucleic acid (preferably DNA) and said nucleic acid will be operably linked to a promoter which is functional in the host cell. The promoter and nucleic acid encoding the protein comprising an NLS-region will usually be part of a vector construct which may also contain signals for termination of transcription, a selectable marker and/or origins of replication functional in the host cell and/or in another cell type (e.g. *E.coli*) so that the vector may be manipulated and grown in the other cell type.

Where an NLS-region sequence contains substitutions, deletions or insertions as described above the alterations to the sequence may be made by manipulation of the nucleic acid sequence to alter the relevant codon(s). This can be achieved by a number of well known standard techniques, e.g. site directed mutagenesis.

Various vectors of this type are described in the Examples herein, and further vectors may be made by those of skill in the art in accordance with routine practice in molecular biology.

In a separate embodiment, the invention also provides a method of directing the location of a protein in a cell to the nucleus which comprises modifying said protein such that is comprises an NLS-region as defined above and, in the case of a DP-3 derived NLS, sufficient C-terminal residues thereof of a DP-3 protein to provide a functional nuclear localisation signal (NLS).

Such a method may be used to modify a DP-protein which does not normally comprise an E region so that the DP-protein (e.g. DP-1 or DP-2 does localise to the nucleus. This can be used to study the function of such DP proteins. These proteins are novel and thus form a further aspect of the invention. Desirably the NLS used to modify a DP-protein is a DP-3 derived NLS.

E2F proteins, particularly E2F-4 and E2F-5 which lack an NLS, may also be modifed by an NLS of the invention. Desirably the NLS used to modify an E2F-protein is an E2F-1-derived NLS.

Modification of such proteins will usually be achieved through the use of recombinant DNA techniques, e.g. using nucleic acid encoding an NLS-region sequence and splicing it to or into nucleic acid encoding the protein of interest. The recombinant nucleic acid may be introduced into an expression vector in a manner analogous to that described above and the vector introduced into a suitable host cell, e.g. a host cell in which a promoter operably linked to the recombinant DNA coding sequence is capable of driving expression of the DNA. Suitable cell types include those described above.

The present invention also comprises an assay for a putative antagonist of cell cycle progression which comprises:
a) expressing in a cell (i) a DP transcription factor or a portion thereof sufficient to form a heterodimer with an E2F transcription factor, wherein said DP transcription factor is unable to localise in the nucleus and (ii) an E2F transcription factor or portion thereof sufficient to form a heterodimer with the DP transcription factor or portion thereof and direct localisation of said heterodimer to the nucleus; and
b) determining the degree of nuclear localisation of said heterodimer in the presence and absence of a putative antagonist of cell cycle progression in order to determine whether or not said putative antagonist is an antagonist of the cell cycle.

The assay may be performed under conditions and within cell types as described above for the assay of an NLS-region regulator, and candidate regulators include those described above for the other assays of the invention.

In this assay, a preferred DP transcription factor is DP-1, particularly mammalian DP-1, e.g. rodent or primate, e.g. human. The sequences of human and mouse DP-1 are shown in SEQ ID Nos. 10 and 11 respectively. A preferred E2F is E2F-1, particularly mammalian E2F-1 (SEQ ID No. 12), respectively e.g. rodent or primate, e.g. human.

Where a portion of an E- DP transcription factor is used in such an assay, it may be of any size which is capable of forming a heterodimer with an E2F transcription factor. Portions of from 40 to 400, preferably 60 to 200 amino acids may be made by routine recombinant DNA techniques and tested in systems analogous to those described above and below in the accompanying examples for their ability to function as required. The portions of the DP protein will generally include substantially all or most of the domain found at amino acids 160 to 220 in DP-1 which is responsible for dimerisation with E2F-1. Where a portion of an E2F transcription factor sufficient to form a heterodimer with the DP transcription factor is used, this may also be made and tested as described above for the portion of the DP factor, and preferably is within the same size ranges and also comprises substantially all or most of the heterodimerisation domain.

The following examples illustrate the invention.

### Example 1: The proteins encoded by the spliced variants of DP-3 have distinct intracellular distributions.

The DP-3 gene gives rise to a number of distinct proteins resulting from alternative splicing of its RNA (Ormondroyd *et al*., 1995). Since the DNA binding and transcription activation properties of the DP-3 variants, referred to as α, β, γ and δ, are not significantly different (Ormondroyd et al., 1995) we considered that the variation within the DP-3 coding sequence may influence other properties of the proteins, such as their biochemical properties. We therefore compared the biochemical extraction properties of β and δ, which constitute E- and E+ forms respectively, after sequential treatment with increasing salt concentration and monitoring the levels of protein extracted from transfected COS7 cells.

COS7 cells were trasfected with plasmids carrying the full length coding sequences of DP-3 α, β, γ and δ (Ormondroyd *et al*., 1995) which were cloned into pG4mpoliII (Webster *et al*., 1989) under the control of the SV40 early promoter. pG4DP-3αΔE mutant was constructed by substituting a Bsgl fragment from DP-3β (E-minus) into DP-3α. A number of other vectors made in connection with other examples are descirbed here for the sake of brevity: The luciferase expression vector pGL-2 was supplied by Promega, and pGL-E vector derived from pGL-2 by an inframe insertion of a 54 bp Xba1 fragment encoding the 16 amino acid residue E region in a single Xba1 site in the luciferase coding region. To generate pGL-Eb, a PCR fragment was amplified using E5-X (5'GCTCTAGAGCCCAGTATAGA-3' (SEQ ID NO: 14)) and E3-X (5'-GCTCTAGATGTCTCAAGCCTTTCCC-3' (SEQ ID NO: 15)) as primers, pG4DP-3α (Ormondroyd *et al.,* 1995) as the template and cloned into the single Xba1 site in pGL-2. pG4-DP-1 has been already described (Bandara *et al.,* 1993) and pRcCMV-HAE2F1 (Krek *et al.*, 1994), expressing HA-tagged human E2F-1 was a gift of Dr W Krek. pCMV-DP-1/NLS was made by inserting a fragment containing the Bel 1 bi-partite NLS (amino acid residue 194 to 227) amplified by PCR into the Kpn1 site (residue 327) of the DP-1 cDNA in pG4-DP-1. The nature of all the constructions were confirmed through sequence analysis.

The cells were grown in Dulbecco's modified Eagle's medium supplemented with 10% foetal calf serum (FCS). Cells were transfected by the liposome-mediated method, using the Lipofectin reagent (Gibco BRL) and according to manufacturer's recommendations. Sixty hours after transfection, cells were lysed in ice cold low salt buffer (LSB; 10mM Tris-HCl pH 8, 7.5mM SO4(NH₄)₂, 1mM EDTA, 0.025% NP-40) by using 0.2 ml of LSB per 6-cm-diameter dish. Lysates were incubated in ice for 5 min, and centrifuged at 3000 rpm for 3 min. The resulting pellets were resuspended in 0.2 ml of high salt buffer (HSB; 50mM Tris-HCl pH 8, 150mM NaCl, 5mM EDTA, 0.5% NP-40) and centrifuged at 10,000 rpm for 5 min. Both buffers, LSB and HSB, were supplemented with protease inhibitors and 1mM dithiothreitol. The insoluble material contained in the pellets of the last centrifugation were resuspended in 0.2 ml of SDS-sample buffer.

Usually, about 5% of the different fractions was used in immunoblotting. Samples were separated on a 10% SDS-polyacrylamide gel and transferred to nitrocellulose membranes. The membrane was blocked with 5% dried milk powder in PBS for 1 h, anti-DP-3 antibody (1:200, rabbit serum) was added and incubated for additional 1 h at room temperature. After three washes in PBS with 0.2% Tween-20, the blot was incubated with alkaline phosphatase-conjugated goat anti-rabbit IgG (1:7500, Promega) for 1 h at room temperature, washed three times in PBS-0.2% Tween 20 and developed. Anti serum 7.5, raised against a peptide containing DEEDEEEDPSSPE (SEQ ID NO: 16) derived from DP-3, was used in the immunoblotting experiments.

The initial treatment with low salt (0.01M) releases mostly soluble cytoplasmic proteins, the high salt (0.5M) both nuclear and cytoplasmic, the insoluble material remaining being collected in fraction designated P. When cells expressing the β variant were treated according to this regime and the levels of β monitored by immunoblotting, it was found to be present throughout the fractions, being moderately enriched in the low salt fraction. In contrast, when cells expressing δ were treated in a similar fashion, the δ protein was far more enriched in the P fraction. Thus, the extraction properties of β and δ are different, and the E region (the only difference between β and δ proteins) is responsible for these differences.

It was possible that the differences in biochemical properties reflected distinct intracellular distributions of the DP-3 proteins. To test this idea we expressed each of the variants in COS7 cells and determined their intracellular location by immunostaining using anti-DP-3 7.2, an antiserum useful for this purpose since it only recognises the exogenous DP-3 protein. For the immunofluorescences, cells were grown on coverslips in 3 cm diameter dishes. When either the α, β, γ or δ variant was expressed in COS7 cells, their intracellular distribution fell into two distinct categories: α and δ accumulated in nuclei whereas β and γ were distributed throughout the cytoplasm with a low level staining in nuclei. Although the α and δ proteins were exclusively nuclear, within a transfected culture of asynchronous cells minor variation was apparent in the distribution of β and γ proteins. For example, β and γ were usually present at higher levels in the cytoplasm relative to nuclei although occasional cells (less than 5% of transfected cells) were seen in which the proteins were present at similar levels in both the nucleus and the cytoplasm, a possible explanation for these observations being suggested later. In summary, these data establish that the differences in protein sequence between the variants influences their intracellular distribution. Specifically, the presence of the E regions in α and δ, but not β and γ, correlates with the ability of the protein to efficiently accumulate in nuclei.

The immunofluorescence was performed as follows.
Transfected cells were fixed in 4% formaldehyde, rinsed and permeabilized in phosphate-buffered saline (PBS) containing 1% Triton X-100. Fixed cells were blocked in PBS containing 1% FCS, incubated with the primary antibodies diluted in PBS-1% FCS for 30 min at room temperature, washed three times with PBS and incubated with the secondary antibodies diluted in PBS-10% FCS for 30 min at room temperature.
After a final wash with PBS, the coverslips were mounted on slides using Citofluor and examined with a Zeiss microscope. Magnification was 630x unless otherwise indicated.

As primary antibodies we used a rabbit polyclonal serum raised against a DP-3 specific peptide common to all the DP-3 variants called 7.2, a rabbit polyclonal serum which detects luciferase (Promega), a DP-1 antiserum (098) raised against a C-terminal peptide in DP-1 and the anti-HA monoclonal antibody 12CA5 (BabCO). Secondary antibodies were goat anti-rabbit IgG conjugated to fluorescein isothiocyanate (1:200, FITC) and goat anti-mouse IgG conjugated to tetramethylrhodamine isothiocyanate (1:200, TRITC) (Southern Biotechnology Associates Inc). Antipeptide serum 7.2 was raised against the sequence VALATGQLPASNSHQ (SEQ ID NO: 17) common to all DP-3 proteins.

### Example 2: The E region is necessary for nuclear localization.

Since the only difference between the β and δ protein is the 16 amino acid residue E region, the E region must be necessary for the nuclear accumulation of δ. To test this idea, we removed the E region from the α variant (which like δ accumulates in nuclei) to create αΔE, and compared the intracellular distribution of the mutated protein to that of wild-type α by immunofluorescence in transfected COS7 cells as described above. The results indicated that in the absence of the E region the intracellular distribution of αΔE was altered to one which resembled the distribution of β since it failed to efficiently accumulate in nuclei. These data support the implications from the previous studies on a requirement for the E region in efficient nuclear accumulation, and thus suggest that it may function as or contribute to a nuclear localization signal (NLS).

### Example 3: An extended E region functions as a nuclear localization signal.

An NLS can be experimentally defined by its deletion causing a loss of nuclear accumulation or by transferring the phenotype to a non nuclear protein. The previous results indicate that the properties of the E region are compatible with the first statement. To address the second, we attached the E region or an extended E region containing an additional 8 residues from the C-terminal boundary, onto luciferase (see Example 1 above for plasmid constructions).

When expressed in COS7 cells, wild-type luciferase was distributed throughout the cell, being marginally more abundant within the cytoplasm; the protein had a very similar distribution in all cells expressing wild-type luciferase. The insertion of the E region (pGL-E) did not significantly alter the distribution of the luciferase protein. However, when an additional 8 residues was inserted (pGL-Eb) nuclear accumulation became far more efficient. Thus, the E region together with additional residues located further on from the C-terminal boundary is necessary for efficient nuclear accumulation.

Together, these data suggest that the E region is necessary but not sufficient for the nuclear accumulation phenotype, and thus the 16 residue sequence is unlikely to contain an autonomous nuclear localization signal. Rather, the E region functions in a co-operative fashion with an additional part of the protein located at the C-terminal boundary of the E region to confer nuclear accumulation. In this respect, the insertion of the E region may produce a bi-partite nuclear localization signal characteristic of many eukaryotic nuclear proteins, such as nucleoplasmin (Dingwall and Laskey, 1991).

### Example 4: The E region is encoded by an alternatively spliced exon.

Although it was very likely that the presence of the E region is regulated by alternative splicing, it was not clear whether a discrete exon encoded the 16 amino acid residues. To clarify this question we isolated the DP-3 gene and characterised its genomic organization across the region encoding the E sequence. For this, a genomic library prepared from murine embryonic stem cells was screened with the DP-3 cDNA, positive clones isolated and thereafter the relationship between genomic and cDNA sequence established.

A λGEM12 genomic library prepared from embryonic stem cell line SV129D3 was plated (approximately 10⁶ pfu) and transferred to Hybond N (Amersham International). Filters were hybridised in QuikHyb solution (Stratagene) at 65°C with a ³²P labelled mouse DP-3α cDNA (Ormondroyd *et al,* 1995). A positive genomic clone which contained the genomic E region was identified via southern blotting using a radiolabelled oligonucleotide antisense to the E region (358-407 bp DP-3α). A genomic fragment containing the E exon was then cloned into pBluescript (pBS, Stratagene) and sequenced using a Sequenase version 2.0 kit (UBS). Oligonucleotides for PCR and sequencing were made from E+ mouse DP-3 cDNA sequences (Ormondroyd *et al*, 1995). Oligonucleotide sequences were as follows: 5' of E region, 7.16S; 5' CACCCGCAATGGTCACT-3'(SEQ ID NO: 18), 3' of E region, 7.17A; 5'-ATGTCTCAAGCCTTTCCC-3' (SEQ ID NO: 19), 5' end of E region E1-S; 5'-GATAGAAAACGAGCTAGAG-3'(SEQ ID NO: 20), 3' end of E region, E2-A; 5'-TTCTGAGAAATCAGAGTCTA-3'(SEQ ID NO: 21).

The analysis indicated that the 16 residues which constitute the E region are indeed encoded by a single 48 bp exon. Conventional splice acceptor and donor sites exist for the boundaries of the E exon which, in turn, lead into two large introns and, subsequently, exon sequence encoding the surrounding DP-3 protein. This isolation and characterisation of the DP-3 gene indicated that the E region is encoded by a discrete alternatively spliced exon. This is illustrated further in Figure 1.

### Example 5: DP-1 lacks an autonomous nuclear localization signal.

A comparison of the E region of DP-3 with the same region of DP-1 indicated that DP-1 lacks a domain analogous to E (Ormondroyd *et al*, 1995). Furthermore, extensive searches to isolate alternatively spliced DP-1 mRNAs have so far failed and thus we investigated the intracellular location of exogenous DP-1 when expressed in COS7 cells, using methods essentially as described above.

The DP-1 protein had a similar distribution to the β and γ (E- minus) forms of DP-3, since it was located throughout the cytoplasm with occasional low level staining in nuclei, such a result being entirely compatible with the absence of the E region. The absence of DP-1 in nuclei was due to the lack of a NLS since the exogenous DP-1 could efficiently accumulate in nuclei after attaching a foreign nuclear localization signal (NLS), the bi-partite signal taken from the Bel 1 protein (Chang et al., 1995). These data suggest that DP-1 is not actively retained in the cytoplasm but rather its cytoplasmic location is passive.

### Example 6: E2F-1 can recruit DP-1 and cytoplasmic DP-3 proteins to nuclei.

The result of Example 5 suggests that the cytoplasmic location of exogenous DP-1 is passive. We reasoned that in the absence of an autonomous NLS a possible mechanism to promote the nuclear accumulation of DP-1 may involve an interaction with its physiological partner, namely the E2F-1 protein. To test this idea, we studied the location of the E2F-1 protein in COS7 cells and thereafter the effect of co-expressing E2F-1 and DP-1 in the same cells.

An E2F-1 protein tagged at its N-terminal with a haemagglutinin (HA) epitope and visualised by immunostaining with an anti-HA monoclonal antibody was exclusively nuclear. To assess the influence of E2F-1 on DP-1, both proteins were co-expressed and their intracellular distribution determined by double immunostaining with anti-HA monoclonal antibody and rabbit anti-DP-1. Neither the fluorescein-congugated anti-rabbit immunoglobulin or rhodamine-congugated anti-mouse immunoglobulin cross-reacted with the anti-HA monoclonal antibody or the rabbit anti-DP-1 respectively.

There was a striking difference in the distribution of DP-1 upon co-expression of E2F-1: cells expressing the E2F-1 protein contained nuclear DP-1, in contrast to its cytoplasmic location in the absence of E2F-1. In the rare exceptions where the transfected cells expressed only DP-1 (about 1% of total transfected population) the exogenous DP-1 was cytoplasmic. These data strongly suggest that upon forming a DP-1/E2F-1 heterodimer, E2F-1 has a dominant influence on recruiting DP-1 to a nuclear location.

We assessed if E2F-1 had a similar effect on DP-3β and αΔE. Co-expression of DP-3 β or αΔE with E2F-1 resulted in nuclear recruitment. The presence of DP-1 or DP-3β in nuclei is likely therefore to be dependent upon an interaction with the appropriate E2F heterodimeric partner which subsequently causes the efficient nuclear accumulation of DP proteins.

### Example 7: E2F-1 contains an NLS.

The abilitiy of E2F-1 to recruit DP-1 to the nucleus was investigated further to identify the E2F-1 NLS. Various experiments are used for this purpose. Deletion mutants of E2F-1 are made and are tested for their ability to recruit DP-1 to the nucleus. Experiments indicate that the NLS of E2F-1 (SEQ ID NO. 12) is located at residues 85-91.

### Discussion: Part A: Summary.

The transport of macromolecules between the cytoplasm and nucleus is mediated in both directions by supramolecular structures which span the nuclear envelope called the nuclear pore complexes (NPCs). Although small macromolecules (less than 40-60kD) can diffuse through NPCs, karyophillic proteins of any size are imported by a selective two-step mechanism which is energy dependent (Fabre and Hurt, 1994; Melchior and Gerace, 1995). Active transport of proteins into the nucleus is dependent upon short stretches of amino acid residues, known as nuclear localization signals (NLS) and, although consensus NLS sequences have been difficult to define, they frequently consist of clusters of basic residues which may be continuous or bi-partite in nature (Dingwall and Laskey,1991; Boulikas, 1993).

Since transcription factors exert their effects on gene expression within the nucleus, it is possible that their activity could be regulated through a control of intracellular location. Mechanisms have been described which influence nuclear accumulation in response to a specific signal, such as direct post-translational modification of the transcription factor, dissociation of an inhibitory subunit which masks the NLS and interaction with a nuclear localizing protein (Whiteside and Goodbourn, 1993). Well documented examples occur in the NF-κB/Rel family of proteins, where proteolytic cleavage of a cytoplasmic precursor or an interaction with cytoplasmic IκB and related proteins controls nuclear accumulation of the functional transcription factor (Siebenlist *et al*., 1995; Norris and Manley, 1995). The glucocorticoid receptor is held in the cytoplasm by virtue of an interaction with heat shock protein 90, and hormone binding widely believed to promote nuclear entry by dissociating the receptor -hsp90 complex (Evans, 1988). In this study, we have documented for the first time mechanisms mediated at the level of intracellular location which influence the nuclear accumulation of the E2F heterodimer.

### Part B: An alternatively spliced nuclear localization signal in the E2F transcription factor.

The E2F transcription factor plays an important role in integrating cell cycle progression with transcription (Nevins, 1992; La Thangue, 1994; Müller, 1995; Weinberg, 1995). In physiological E2F members of two distinct families of proteins, DP and E2F, interact as DP/E2F heterodimers (Bandara *et al*., *1993),* with the functional consequences being co-operative DNA binding, pocket protein binding and transcriptional activation (Bandara *et al*., 1993; Helin *et al.,* 1993a; Krek *et al*., 1993). A number of different levels of control are known to be exerted upon the E2F heterodimer, such as binding and transcriptional repression by the pocket proteins (Helin *et al.,* 1993b; Flemington *et al.,* 1993), phosphorylation by cdk complexes (Krek *et al*., 1994; 1995) and transcriptional activation by MDM2 oncoprotein (Martin *et al*., 1995). Here, we have described an additional mechanism of control in regulating the activity of E2F mediated at the level of intracellular location. Specifically, our data show that two alternative mechanisms exist which control the nuclear accumulation of the DP/E2F heterodimer regulated, firstly, by alternative splicing and, secondly, subunit composition of the heterodimer.

These conclusions relate to previous observations made on the DP-3 gene which encodes a number of discrete mRNAs that arise through alternative splicing. (Ormondroyd *et al.,* 1995). One of these processing events determines whether the E region is incorporated in the protein. Here, we show that the E region is encoded by an alternatively spliced exon which, together with an additional C-terminal extension, can confer efficient nuclear accumulation. The E region therefore contributes to a nuclear localization signal.

Interestingly, comparison of the sequence of the sixteen amino acid residues within the E region to other previously defined NLSs suggests a closer resemblance to a bi-partite NLS rather than the NLS characteristic of SV40 large T antigen (Dingwall and Laskey, 1991). Although there is some similarity to the SV40 large T antigen-like NLS, neither the sequence nor the functional properties of the E region completely satisfy the requirements for this type of NLS (Boulikas, 1993; 1994). For example, the consensus core sequence for an SV40 large T-like motif is likely to consist of at least four arginine and lysine residues, whereas the cluster within the E region consists of three basic residues. Secondly, acidic residues are rarely included within the signal sequence, yet the E region cluster contains an aspartate residue embedded within it.

Functional evidence for this idea was obtained by determining if the E region is necessary and sufficient for nuclear accumulation. Although necessary in the context of wild-type DP-3 sequence, alone the E region was not sufficient to confer onto a non-nuclear resident efficient nuclear accumulation, but rather required an additional region located immediately C-terminal of the E region. This sequence, together with the cluster of basic residues within the E region, has a similar arrangement and characteristics for a bi-partite NLS namely, two basic clusters of amino acid residues separated by a spacer region (Dingwall and Laskey, 1991; La Casse and Lefebvre, 1995). In the DP-3 variants β and γ which lack in the E region, the N-terminal half of the bi-partite signal is removed by the splicing of the E exon.

The role of alternative splicing as a mechanism for generating protein isoforms with different functional properties has been widely described. The inclusion of sequences which function as NLSs has been reported in several cases, such as in the nuclear mitotic apparatus (NuMA) protein (Tang *et al*., 1994), CaM kinase (Srinivasan et al., 1994) and deoxynucleotidyl transferase (Bentolila *et al*., 1995). An interesting situation occurs in the Max gene, which encodes a heterodimeric partner for Myc, where Max RNA is alternatively spliced to result in a Max protein truncated at the C-terminus and lacking an NLS (Makela et al., 1992). In contrast to wild-type Max, the truncated Max protein enhances the transformation activity of Myc (Makela *et al;* 1992). Nevertheless, a physiological splicing event which regulates a bi-partite NLS in such a fashion by removing one of the clusters of basic residues is, to our knowledge, novel. Thus, these data define a previously unidentified level of control in the E2F transcription factor and could, more generally, indicate a new mechanism for regulating the activity of bi-partite NLSs through RNA processing.

Although these data establish a dependence upon the E region for nuclear accumulation, they do not distinguish between the possibilities that the E region regulates nuclear entry or export. For example, it is possible that E- variants can. enter and exit nuclei, and that the presence of the E region impedes nuclear export, resulting in a net nuclear accumulation. Such a possibility would be compatible with the altered biochemical extraction properties confired by the E region, which suggested that the E region may be involved in tethering to an insoluble nuclear structure. Interestingly, pRb is believed to be held in the nucleus by a tethering process, a property characteristic of the hypophosphorylated protein and thus potentially important in mediating physiological effects of cell cycle arrest (Mittnacht *et al.,* 1991).

### Part C: Heterodimer formation between DP and E2F family members provides a mechanism for efficient nuclear accumulation.

The DP-3β and γ variants fail to accumulate in nuclei when expressed in COS7 cells, a phenotype which can now be directly attributed to the absence of the E region. The DP-1 protein, which lacks a region analogous to E (Girling *et al*, 1993; Ormondroyd *et al*, 1995), behaved in a fashion predicted for an E-DP variant since exogenous DP-1 protein on COS7 cells had a similar location as the DP-3 E-variants.

The distribution of the E- DP variants, which are predominantly cytoplasmic, could result from one of several scenarios. For example, passive diffusion may occur such that at equilibrium the proteins are more abundant within the cytoplasm. Alternatively, the proteins may have a weak NLS which fails to efficiently target them to nuclei, a possibility consistent with the E- variants still possessing one half of the bi-partite NLS and observations made on the nucleoplasmin NLS where elimination of one half of the bi-partite signal does not completely abolish nuclear accumulation (Robbins *et al*., 1991). Finally, it is also possible that the cytoplasmic pattern results from an active retention mechanism. However, this latter possibility is unlikely since a heterologous NLS was sufficient to confer a nuclear accumulation phenotype.

We reasoned that there must be physiological mechanisms which promote the efficient nuclear accumulation of DP-1 given that the endogenous DP-1 is nuclear (data not shown). We therefore tested whether formation of a DP/E2F heterodimer was involved in such a mechanism, experiments which indicated that co-expression of E2F-1 recruited E- DP proteins to nuclei, and thus heterodimerization with an appropriate E2F family member is likely to be sufficient to promote nuclear accumulation. Mechanistically, the nuclear accumulation of E- DP variants upon an interaction with E2F-1 may occur if E2F-1 is tethered within the nucleus and, upon interacting with DP variants, causes their retention in the nucleus. Alternatively, the interaction with E2F-1 may occur within the cytoplasm and the physical interaction with E2F-1 be responsible for delivering E- DP variants to the nucleus. Overall, these data suggest two distinct mechanisms for the nuclear accumulation of DP proteins, one dependent on the presence of an intrinsic sequence in the protein and the other on an interaction with the appropriate E2F partner.

The fact that heterodimer formation can promote nuclear accumulation provides a likely explanation for the small proportion of COS7 cells which contain exogenous nuclear β protein. We suggest in such cells that β has a nuclear location by virtue of an interaction and heterodimer formation with endogenous E2F proteins.

### Part D: Physiological implications

A mechanism through which nuclear accumulation is dependent upon heterodimerization has a number of important implications for the regulation of functional E2F transcription factor, that is, the DP/E2F heterodimer. For example, it would favour the presence of DP/E2F heterodimers, the physiological form involved in transcriptional activation (Bandara *et al*., 1993; Helin *et al*., 1993b; Krek *et al*., 1993), in nuclei perhaps preventing some non-specific and/or undesirable interactions occurring. It may, in addition, provide a mechanism whereby the induction of nuclear DP/E2F heterodimers is dependent on a rate limiting E2F partner. Indeed, the expression of the E2F-1 gene is known to be under cell cycle control, in contrast to DP-1 which in some cell types is constitutively expressed during the cell cycle (Slansky *et al*., 1993). In such a model, although DP-1 is expressed its contribution to transcriptional activation in the context of the DP/E2F heterodimer during the cell cycle will be strictly dependent upon the levels of E2F-1.

We have established that the E region of DP proteins is required for nuclear accumulation, and that it likely functions as a bi-partite nuclear localization signal. Although this situation is novel, as yet we do have to understand the role that this mechanism plays in physiological E2F and the regulation of cell cycle progression. It is possible, we suggest, the E+ variants of DP proteins function in an analogous fashion as E2F-1 for DP-1 to recruit proteins capable of interacting with E+ variants but which lack an autonomous nuclear localization signal.

In conclusion, this study has highlighted a new and unexpected mechanism of control in regulating the activity of the E2F heterodimer. Specifically, nuclear accumulation is dramatically influenced by two distinct levels of control: alternative splicing of an exon which contributes to a nuclear localization signal and the subunit composition of the E2F heterodimer. It is likely that this control plays an important role in regulating the activity of the E2F transcription factor and hence cell cycle progression.

### REFERENCES

Bandara, L.R. and La Thangue, N.B. (1991). Nature **351**: 494-497.
Bandara, L.R., *et al* (1991). *Nature* **352:** 249-251.
Bandara, L.R., Buck, V.M., Zamanian, M., Johnston, L.H. and La Thangue, N.B. (1993). *EMBO J.* **12,** 4317-4324.
Bandara, L.R., Lam, E.W.-F., Sørensen, T.S., Zamanian, M., Girling, R. and La Thangue, N.B. (1994). *EMBO J*. **13**, 3104-3114.
Beijersbergen, R.L., Kerkhoven, R.M., Zhu, L., Carlee, L., Voorhoeve, P.M. and Bernards, R. (1994). *Genes. Dev*. **8,** 2680-2690.
Beijersbergen, R.L., *et al* (1995). *Genes Dev*. **9:** 1340-1353.
Bentolila, L.A. *et al* (1995). *EMBO J.* **14:** 4221-4229.
Boulikas, T. (1993). *Crit. Rev. Eukar. Gene Expr*. **3:** 193-227.
Boulikas, T. (1994). *J. Cell Biochem*. **55:** 32-38.
Boulikas, T. (1993). *Crit. Rev. Eukar. Gene Expr.* **3:** 193-227.
Buck, V., Allen, E.K., Sørensen, T., Bybee, A., Hijmans, E.M., Voorhoeve, P.M., Bernards, R. and La Thangue, N.B. (1995). *Oncogene,* **11,** 31-38.
Chang, J., Lee K.J., Jang, K.L., Lee, E.K., Baek, G. H. and Sung, Y.C. (1995). *J. Virology* **69:** 801-808.
Chellappan, S.P. *et al* (1991). *Cell* **65:** 1053-1061.
Chellappan, S., *et al* (1992). *Proc. Natl. Acad. Sci. USA* **89**: 4549-4553.
Dingwall, C. and Laskey, R. (1991). *Trends. Biochem.* Sci **16:** 478-481.
Dowdy, S.F. *et al* (1993). *Cell* **73:** 499-511.
Evans, R.M. (1988). *Science* **240:** 889-895.
Ewen, M.E. *et al* (1993). *Cell* **73:** 487-497.
Fabre, E. and Hurt, E.C. (1994). *Cur. Op. Cell Biol*. **6:** 335-342.
Flemington, E.K., Speck, S.H. and Kaelin, W.G. (1993). *Proc. Natl. Acad. Sci. USA.* **90,** 6914-6918.
Ginsberg, D., Vairo, G., Chittenden, T., Xiao, Z.-X., Xu. G., Wydner, K.L., DeCaprio, J.A., Lawrence, J.B. and Livingston, D.M. (1994). *Genes. Dev.* **8,** 2665-2679.
Girling, R., Partridge, J.F., Bandara, L.R., Burden, N., Totty, N.F., Hsuan, J.J. and La Thangue, N.B. (1993). *Nature* **362,** 83-87.
Girling, R., Bandara, L.R., Ormondroyd, E., Lam, E.W.-F., Kotecha, S., Mohun, T. and La Thangue, N.B. (1994). *Mol. Biol. Cell.* **5,** 1081-1092.
Heibert, S.W., Chellappan, S.P., Horowitz, J.M. and Nevins, J.R. (1992). *Genes Dev*. **6,** 177-185.
Helin, K., Lees, J.A., Vidal, M., Dyson, N., Harlow, E. and Fattaey, A. (1992). *Cell* **70,** 337-350.
Helin, K., Wu, C.-L., Fattaey, A.R., Lees, J.A., Dynlacht, B.D., Ngwu, C. and Harlow, E. (1993b). *Genes Dev*. **7,** 1850-1861.
Helin, K., Harlow, E. and Fattaey, A.R. (1993a). *Mol. Cell. Biol*. **13:** 6501-6508.
Hijmans, E.M. *et al* (1995). *Mol. Cell. Biol.* **15:** 3082-3089. Hinds, P.W., Mittnacht, S., Dulic, V., Arnold, A., Reed, S.L. and Ivey-Hoyle, M., Conroy, R., Huber, H.E., Goodhart, P.J., Oliff, A. and Heimbrook, D.C. (1993). *Mol. Cell. Biol.* **13,** 7802-7812.
Ivey-Hoyle, M. *et al* (1993). *Mol. Cell. Biol.* **13:** 7802-7812.
Johnson, D.G. *et al* (1994). *Proc. Natl. Acad. Sci. USA* **91:** 12823-12827.
Jooss, K. *et al ( 1995). Oncogene* **10:** 1529-1536.
Kaelin, W.G., Krek, W., Sellers, W.R., DeCaprio, J.A., Ajchenbaum, F., Fuchs, C.S., Chittenden, T., Li, Y., Farnham, P.J., Blanar, M.A., Livingston, D.M. and Flemington, E.K. (1992). *Cell* **70,** 351-364.
Krek, W., Ewen, M.E., Shirodkar, S., Arany, Z., Kaelin, W.G. and Livingston, D.M. (1994). *Cell* **78,** 161-172.
Krek, W., Livingston, D.M. and Shirodkar, S. (1993). *Science* **262,** 1557-1560.
Krek, W. *et al* (1995). *Cell* **83:** 1149-1158.
La Casse, E.C. and Lefebvre, Y.A. (1995). *Nuc. Ac. Res.* **23:** 1647-1656.
La Thangue, N.B. (1994). *Trends Biochem. Sci.* **19,** 108-114.
Lees, J.A., Saito, M., Vidal, M., Valentine, M., Look, T., Harlow, E., Dyson, N. and Helin, K. (1993). *Mol. Cell. Biol.* **13,** 7813-7825.
Lees, E., Faha, B., Dulic, V., Reed, S.I. and Harlow, E. (1992). *Genes Dev.* **6,** 1874-1885.
Makela, T.P. *et al* (1992). *Science* **256:** 373-377.
Martin, K. *et al* (1995). *Nature* **375:** 691-694.
Melchior, F. and Gerace, L. (1995). *Cur. Op. Cell Biol*. **7:** 310-318.
Mittnacht, S. and Weinberg, R.A. (1991). *Cell* **65:** 381-393.
Morris, J.D. *et al* (1993). *Oncogene* **8:** 893-898.
Müller, R. (1995). *Trends Genet.* **11:** 173-178.
Nevins, J.R. (1992). *Science* **258,** 424-429.
Norris, J.L. and Manley, J.L. (1995). *Inducible Gene Expression,* Vol. 2, 243-265. P.A. Baeuerle, Ed. Birkhaüser Boston.
Ormondroyd, E., de la Luna, S. and La Thangue, N. (1995) *Oncogene* **11,** 1437-1446.
Robbins, J. *et al* (1991). *Cell* **64:** 615-623.
Sardet, C. *et al* (1995). *Proc. Natl. Acad. Sci. USA* **92:** 2403-2407.
Schwarz, J.K., Devoto, S.H., Smith, E.J., Chellappan, S.P., Jakoi, L. and Nevins, J.R. (1993). Interactions of the p107 and Rb proteins with E2F during the cell proliferation response. *EMBO J.* **12:** 1013-1020.
Shan, B., Zhu, X., Chen, P.L., Durfee, T., Yang, Y., Sharp, D. and Lee, W.H. (1992). *Mol. Cell. Biol.* **12,** 5620-5631.
Sherr, C.J. (1993). Mammalian G1 cyclins. *Cell* **73:** 1059-1065.
Siebenlist, U. *et al* (1995). *Inducible Gene Expression,* Vol. 1, 93-141. P.A. Baeuerle, Ed. Birkhaüser Boston.
Slansky, J.E. *et al* (1993). *Mol. Cell. Biol*. **13:** 1610-1618.
Srinivasan, M. *et al* (1994). *J. Cell. Biol.* **126:** 839-852.
Tang, T.K *et al* (1994). *J. Cell Sci.* **107:** 1389-1402.
Vairo, G. *et al* (1995). *Genes Dev.* **9:** 869-881.
Webster, N.J.G., Green, S., Tasset, D., Ponglikitmongkol, M. and Chambon, P. (1989). *EMBO. J.* **8:** 1441-1446.
Weinberg, R.A. (1995). *Cell* **81:** 323-330.
Whiteside, S.T. and Goodbourn, S. (1993). *J. Cell Sci.* **104:** 949-955.
Wolf, D.A. *et al* (1995). *Oncogene* **10:** 2067-2078.
Wu, C.-L., Zukerberg, L.R., Ngwu, C., Harlow, E. and Lees, J.A. (1995). *Mol. Cell. Biol*. **15,** 2536-2546.
Zamanian, M. and La Thangue, N.B. (1992). *EMBO J.* **11,** 2603-2610.
Zamanian, M. and La Thangue, N.B. (1993). *Mol. Biol. Cell.* **4**,389-396.
Zhang, Y. and Chellappan, S. (1995). *Oncogene,* **10,** 2085-2093.
Zhu, L., Van der Heurel, S., Helin, K., Fattaey, A., Ewen, M., Livingston, D., Dyson, N. and Harlow, E. (1993). *Genes Dev.* **7,** 1111-1125.
Zhu, L. *et al* (1995a). *EMBO J.* **14:** 1904-1913.
Zhu, L. *et al* (1995b). *Genes Dev*. **9:** 1740-1752.

## Claims

1. An assay for a putative regulator of cell cycle progression which comprises:
a) expressing in a cell a protein comprising
(i) the E region and sufficient C-terminal residues thereof of a DP-3 protein to provide a functional nuclear localisation signal (NLS), said E region having a sequence: or a variant thereof modified by substitution, insertion or deletion wherein said variant retains NLS activity and wherein modification by substitution is substitution of from 1 to 4 amino acids and modification by insertion or deletion is insertion or deletion of from 1 to 3 amino acids; and
(ii) a marker for nuclear localization fused to (i); and
b) determining the degree of nuclear localization of said protein in the presence and absence of a putative regulator of cell cycle progression in order to determine whether or not said putative regulator is a regulator of the cell cycle.

2. An assay according to claim 1 wherein the NLS comprises the sequence:

3. An assay according to claim 1 or 2 wherein the number of C-terminal residues is from 8 to 20.

4. An assay for a putative regulator of cell cycle progression which comprises:
a) expressing in a cell a protein comprising
(i) the nuclear localisation signal (NLS) of E2F-1, said NLS having a sequence comprising residues 85-91 of SEQ ID NO:12, or a variant thereof modified by substitution, insertion or deletion wherein said variant retains NLS activity and wherein modification by substitution is substitution of from 1 to 4 amino acids and modification by insertion or deletion is insertion or deletion of from 1 to 3 amino acids; and
(ii) a marker for nuclear localization fused to (i); and
b) determining the degree of nuclear localization of said protein in the presence and absence of a putative regulator of cell cycle progression in order to determine whether or not said putative regulator is a regulator of the cell cycle.

5. An assay according to any one of claims 1 to 4 wherein the cell is a yeast, insect or mammalian cell.

6. An assay according to claim 5 wherein the mammalian cell is a primate cell.

7. An assay according to any one of claims 1 to 6 wherein the marker comprises an antigenic determinant bindable by an antibody.

8. An assay according to any one of claims 1 to 6 wherein the marker comprises an enzyme capable of causing a colour change to a substrate.

9. An assay according to any one of claims 1 to 6 wherein the marker comprises a luciferase enzyme.

10. An assay according to any one of claims 1 to 6 wherein the marker comprises a transcription factor or subunit thereof, which transcription factor is capable of activating an indicator gene.

11. An assay according to claim 10 wherein said marker comprises the DNA binding domain (DBD) or the transcriptional activation domain (TAD) of the yeast transcription factor GAL 4, and the indicator gene comprises a GAL 4 promoter.

12. An assay according to claim 11 wherein the indicator gene is chloramphenicol acetyl transferase (CAT) or a luciferase.

13. An assay according to any one of the preceding claims wherein the regulator is a peptide comprising all or part of a sequence which has from 60 to 100% amino acid identity to a portion of the DP-3 E region (SEQ ID NO:9) of the same length.

14. An assay according to any one of the preceding claims wherein the expression of the protein is a transient expression.

15. An assay according to any one of claims 1 to 13 wherein the cell is stably transfected with a construct expressing the protein.

16. An assay according to any one of the preceding claims wherein said putative regulator is an antagonist.

17. A method of directing the location of a protein in a cell to the nucleus which method comprises modifying said protein such that it comprises either:
(i) the E region and sufficient C-terminal residues thereof of a DP-3 protein to provide a functional nuclear localisation signal (NLS), said E region having a sequence: or a variant thereof modified by substitution, insertion or deletion wherein said variant retains NLS activity and wherein modification by substitution is substitution of from 1 to 4 amino acids and modification by insertion or deletion is insertion or deletion of from 1 to 3 amino acids; or
(ii) the nuclear localisation signal (NLS) of E2F-1, said NLS having a sequence comprising residues 85-91 of SEQ ID NO:12, or a variant thereof modified by substitution, insertion or deletion wherein said variant retains NLS activity and wherein modification by substitution is substitution of from 1 to 4 amino acids and modification by insertion or deletion is insertion or deletion of from 1 to 3 amino acids.

18. A method according to claim 17 wherein said protein is a DP-protein which does not normally comprise said E region.

19. A protein which does not normally comprise the E region of a DP-3 whose sequence has been modified to comprise said E region, said E region being the E region and sufficient C-terminal residues thereof of a DP-3 protein to provide a functional nuclear localisation signal (NLS), said E region having a sequence: or a variant thereof modified by substitution, insertion or deletion wherein said variant retains NLS activity and wherein modification by substitution is substitution of from 1 to 4 amino acids and modification by insertion or deletion is insertion or deletion of from 1 to 3 amino acids.

20. An assay for a putative antagonist of cell cycle progression which comprises:
a) expressing in a cell (i) a DP transcription factor or a portion thereof sufficient to form a heterodimer with an E2F transcription factor, wherein said DP transcription factor is unable to localize in the nucleus and (ii) an E2F transcription factor or portion thereof sufficient to form a heterodimer with the DP transcription factor or portion thereof and direct localisation of said heterodimer to the nucleus; and
b) determining the degree of nuclear localization of said heterodimer in the presence and absence of a putative antagonist of cell cycle progression in order to determine whether or not said putative antagonist is an antagonist of the cell cycle.

21. An assay according to claim 20 wherein the DP transcription factor is DP-1.

22. An assay according to claim 20 or 21 wherein the E2F is E2F-1.

## Patentansprüche

1. Test auf einen mutmaßlichen Regulator der Zellzyklus-Progression, Folgendes umfassend:
a) das Exprimieren eines Proteins, das Folgendes umfasst, in einer Zelle:
(i) die E-Region und ausreichend C-terminale Reste davon eines DP-3-Proteins, um ein funktionelles Nucleus-Lokalisierungs-Signal (NLS) bereitzustellen, wobei die E-Region die Sequenz oder eine durch Substitution, Insertion oder Deletion modifizierte Variante davon aufweist, wobei die Variante NLS-Aktivität beibehält und wobei die Modifikation durch Substitution eine Substitution von 1 bis 4 Aminosäuren ist und die Modifikation durch Insertion oder Deletion eine Insertion oder Deletion von 1 bis 3 Aminosäuren ist; und
(ii) einen an (i) fusionierten Marker für die Nucleus-Lokalisierung; sowie
b) das Bestimmen des Grads an Nucleus-Lokalisierung des Proteins in Gegenwart und Abwesenheit eines mutmaßlichen Regulators der Zellzyklus-Progression, um zu bestimmen, oder der mutmaßliche Regulator ein Regulator des Zellzyklus ist oder nicht.

2. Test nach Anspruch 1, worin das NLS die Sequenz umfasst.

3. Test nach Anspruch 1 oder 2, worin die Anzahl der C-terminalen Reste 8 bis 20 beträgt.

4. Test auf einen mutmaßlichen Regulator der Zellzyklus-Progression, Folgendes umfassend:
a) das Exprimieren eines Proteins, das Folgendes umfasst, in einer Zelle:
(i) das Nucleus-Lokalisierungs-Signal (NLS) von E2F-1, wobei das NLS eine Sequenz, welche die Reste 85 bis 91 von Seq.-ID Nr. 12 umfasst, oder eine durch Substitution, Insertion oder Deletion modifizierte Variante davon aufweist, wobei die Variante NLS-Aktivität beibehält und wobei die Modifikation durch Substitution eine Substitution von 1 bis 4 Aminosäuren ist und die Modifikation durch Insertion oder Deletion eine Insertion oder Deletion von 1 bis 3 Aminosäuren ist; und
(ii) einen an (i) fusionierten Marker für die Nukleus-Lokalisierung; sowie
b) das Bestimmen des Grads an Nucleus-Lokalisierung des Proteins in Gegenwart und Abwesenheit eines mutmaßlichen Regulators der Zellzyklus-Progression, um zu bestimmen, ob der mutmaßliche Regulator ein Regulator des Zellzyklus ist oder nicht.

5. Test nach einem der Ansprüche 1 bis 4, worin die Zelle eine Hefe-, Insektenoder Säugetierzelle ist.

6. Test nach Anspruch 5, worin die Säugetierzelle eine Primatenzelle ist.

7. Test nach einem der Ansprüche 1 bis 6, worin der Marker eine Antigen-Determinante ist, die von einem Antikörper gebunden werden kann.

8. Test nach einem der Ansprüche 1 bis 6, worin der Marker ein Enzym umfasst, das eine Farbveränderung eines Substrats hervorrufen kann.

9. Test nach einem der Ansprüche 1 bis 6, worin der Marker ein Luciferase-Enzym umfasst.

10. Test nach einem der Ansprüche 1 bis 6, worin der Marker einen Transkriptionsfaktor oder eine Untereinheit davon umfasst, wobei der Transkriptionsfaktor fähig ist, ein Indikatorgen zu aktivieren.

11. Test nach Anspruch 10, worin der Marker die DNA-Bindungsdomäne (DBD) oder die Transkriptions-Aktivierungsdomäne (TAD) des Hefe-Transkriptionsfaktors GAL 4 umfasst und das Indikatorgen einen GAL 4-Promotor umfasst.

12. Test nach Anspruch 11, worin das Indikatorgen Chloramphenicol-Acetyl-Transferase (CAT) oder eine Luciferase ist.

13. Test nach einem der vorangegangenen Ansprüche, worin der Regulator ein Peptid ist, das die gesamte oder einen Teil einer Sequenz umfasst, die 60 bis 100 % Aminosäure-Identität mit einem Abschnitt der DP-3 E-Region (Seq.-ID Nr. 9) derselben Länge aufweist.

14. Test nach einem der vorangegangenen Ansprüche, worin die Expression des Proteins eine vorübergehende Expression ist.

15. Test nach einem der Ansprüche 1 bis 13, worin die Zelle mit einem das Protein exprimierenden Konstrukt stabil transfiziert wird.

16. Test nach einem der vorangegangenen Ansprüche, worin der mutmaßliche Regulator ein Antagonist ist.

17. Verfahren zum Dirigieren der Position eines Proteins in einer Zelle zum Zellkern,
wobei das Verfahren das Modifizieren des Proteins auf solche Weise umfasst, dass es Folgendes umfasst:
(i) entweder die E-Region und ausreichend C-terminale Reste davon eines DP-3-Proteins, um ein funktionelles Nucleus-Lokalisierungs-Signal (NLS) bereitzustellen, wobei die E-Region eine Sequenz oder eine durch Substitution, Insertion oder Deletion modifizierte Variante davon aufweist, wobei die Variante NLS-Aktivität beibehält und wobei die Modifikation durch Substitution eine Substitution von 1 bis 4 Aminosäuren ist und die Modifikation durch Insertion oder Deletion eine Insertion oder Deletion von 1 bis 3 Aminosäuren ist;
(ii) oder das Nucleus-Lokalisierungs-Signal (NLS) von E2F-1, wobei das NLS eine Sequenz, die die Reste 85 bis 91 von Seq.-ID Nr. 12 umfasst, oder eine durch Substitution, Insertion oder Deletion modifizierte Variante davon aufweist, wobei die Variante NLS-Aktivität beibehält und wobei die Modifikation durch Substitution eine Substitution von 1 bis 4 Aminosäuren ist und die Modifikation durch Insertion oder Deletion eine Insertion oder Deletion von 1 bis 3 Aminosäuren ist.

18. Verfahren nach Anspruch 17, worin das Protein ein DP-Protein ist, das die E-Region normalerweise nicht umfasst.

19. Protein, das normalerweise nicht die E-Region eines DP-3 umfasst, dessen Sequenz so modifiziert worden ist, dass sie die E-Region umfasst, wobei die E-Region die E-Region und ausreichend C-terminale Reste davon eines DP-3-Proteins ist, um ein funktionelles Nucleus-Lokalisierungs-Signal (NLS) bereitzustellen, wobei die E-Region die Sequenz: oder eine durch Substitution, Insertion oder Deletion modifizierte Variante davon aufweist, wobei die Variante NLS-Aktivität beibehält und wobei die Modifikation durch Substitution eine Substitution von 1 bis 4 Aminosäuren ist und die Modifikation durch Insertion oder Deletion eine Insertion oder Deletion von 1 bis 3 Aminosäuren ist.

20. Test auf einen mutmaßliche Antagonisten der Zellzyklus-Progression, Folgendes umfassend:
a) das Exprimieren (i) eines DP-Transkriptionsfaktors oder eines Abschnitts davon, der ausreicht, um ein Heterodimer mit einem E2F-Transkriptionsfaktor zu bilden, worin der DP-Transkriptionsfaktor nicht fähig ist, sich im Zellkern zu lokalisieren, und (ii) eines E2F-Transkriptionsfaktors oder eines Abschnitts davon, der ausreicht, um ein Heterodimer mit dem DP-Transkriptionsfaktor oder einem Abschnitt davon zu bilden und die Lokalisierung des Heterodimers zum Zellkern zu dirigieren, in einer Zelle; und
b) das Bestimmen des Grads an Nucleus-Lokalisierung des Heterodimers in Gegenwart und Abwesenheit eines mutmaßlichen Antagonisten der Zellzyklus-Progression, um zu bestimmen, ob der mutmaßliche Antagonist ein Antagonist des Zellzyklus ist oder nicht.

21. Test nach Anspruch 20, worin der DP-Transkriptionsfaktor DP-1 ist.

22. Test nach Anspruch 20 oder 21, worin das E2F E2F-1 ist.

## Revendications

1. Essai pour un régulateur putatif de la progression d'un cycle cellulaire, qui comprend :
a) l'expression, dans une cellule, d'une protéine comprenant
(i) la région E et suffisamment de résidus C-terminaux d'une protéine DP-3 pour produire un signal fonctionnel de localisation nucléaire (NLS), ladite région E ayant une séquence : ou une variante de celle-ci modifiée par substitution, insertion ou délétion, où ladite variante conserve l'activité de NLS et où une modification par substitution est une substitution de 1 à 4 acides aminés et une modification par insertion ou délétion est une insertion ou délétion de 1 à 3 acides aminés ; et
(ii) un marqueur pour la localisation nucléaire fusionné à (i) ; et
b) la détermination du degré de localisation nucléaire de ladite protéine en présence et en l'absence d'un régulateur putatif de la progression du cycle cellulaire afin de déterminer si ledit régulateur putatif est ou non un régulateur du cycle cellulaire.

2. Essai selon la revendication 1, où NLS comprend la séquence :

3. Essai selon la revendication 1 ou 2, où le nombre de résidus C-terminaux est de 8 à 20.

4. Essai pour un régulateur putatif de la progression d'un cycle cellulaire qui comprend :
a) l'expression, dans une cellule, d'une protéine comprenant
(i) le signal de localisation nucléaire (NLS) de E2F-1, ledit NLS ayant une séquence comprenant les résidus 85-91 de SEQ ID NO:12, ou une variante modifiée par substitution, insertion ou délétion, où ladite variante conserve l'activité de NLS et où la modification par substitution est une substitution de 1 à 4 acides aminés et la modification par insertion ou délétion est une insertion ou délétion de 1 à 3 acides aminés ; et
(ii) un marqueur pour la localisation nucléaire fusionné à (i) ; et
b) la détermination du degré de localisation nucléaire de ladite protéine en présence et en l'absence d'un régulateur putatif de la progression du cycle cellulaire afin de déterminer si ledit régulateur putatif est ou non un régulateur du cycle cellulaire.

5. Essai selon l'une quelconque des revendications 1 à 4, où la cellule est une cellule de levure, d'insecte ou mammalienne.

6. Essai selon la revendication 5, où la cellule mammalienne est un cellule de primate.

7. Essai selon l'une quelconque des revendications 1 à 6, où le marqueur comprend un déterminant antigénique liable par un anticorps.

8. Essai selon l'une quelconque des revendications 1 à 6, où le marqueur comprend une enzyme capable de provoquer un changement de couleur d'un substrat.

9. Essai selon l'une quelconque des revendications 1 à 6, où le marqueur comprend une enzyme luciférase.

10. Essai selon l'une quelconque des revendications 1 à 6, où le marqueur comprend un facteur de transcription ou sous-unité de celui-ci, lequel facteur de transcription est capable d'activer un gène indicateur.

11. Essai selon la revendication 10, où ledit marqueur comprend le domaine liant l'ADN (DBD) ou le domaine d'activation de transcription (TAD) du facteur de transcription de la levure GAL 4, et le gène indicateur comprend un promoteur de GAL 4.

12. Essai selon la revendication 11, où le gène indicateur est la chloramphénicol acétyl transférase (CAT) ou une luciférase.

13. Essai selon l'une quelconque des revendications précédentes, où le régulateur est un peptide comprenant tout ou partie d'une séquence qui a de 60 à 100% d'identité d'acides aminés avec une portion de la région E de DP-3 (SEQ ID NO:9) de la même longueur.

14. Essai selon l'une quelconque des revendications précédentes, où l'expression de la protéine est une expression transitoire.

15. Essai selon l'une quelconque des revendications 1 à 13, où la cellule est stablement transfectée avec une construction exprimant la protéine.

16. Essai selon l'une quelconque des revendications précédentes, où ledit régulateur putatif est un antagoniste.

17. Méthode pour diriger la localisation d'une protéine dans une cellule vers le noyau, laquelle méthode consiste à modifier ladite protéine de façon qu'elle comprenne soit :
(i) la région E et suffisamment de résidus C-terminaux de celle-ci d'une protéine DP-3 pour produire un signal fonctionnel de localisation nucléaire (NLS), ladite région E ayant une séquence : ou sa variante modifiée par substitution, insertion ou délétion, où ladite variante conserve l'activité de NLS et où la modification par substitution est une substitution de 1 à 4 acides aminés et la modification par insertion ou délétion est l'insertion ou délétion de 1 à 3 acides aminés ; ou
(ii) le signal de localisation nucléaire (NLS) de E2F-1, ledit NLS ayant une séquence comprenant les résidus 85-91 de SEQ ID NO:12, ou une variante modifiée par substitution, insertion ou délétion, où ladite variante conserve l'activité de NLS et où la modification par substitution est une substitution de 1 à 4 acides aminés et la modification par insertion ou délétion est l'insertion ou délétion de 1 à 3 acides aminés.

18. Méthode selon la revendication 17, où ladite protéine est une protéine DP qui ne comprend pas normalement ladite région E.

19. Protéine qui ne comprend pas normalement la région E de DP-3 dont la séquence a été modifiée pour comprendre ladite région E, ladite région E étant la région E et suffisamment de résidus C-terminaux d'une protéine DP-3 pour produire un signal fonctionnel de localisation nucléaire (NLS), ladite région E ayant une séquence : ou sa variante modifiée par substitution, insertion ou délétion, où ladite variante conserve l'activité de NLS et où une modification par substitution est une substitution de 1 à 4 acides aminés et une modification par insertion ou délétion est une insertion ou délétion de 1 à 3 acides aminés.

20. Essai pour un antagoniste putatif de la progression du cycle cellulaire qui comprend :
a) l'expression, dans une cellule, (i) d'un facteur de transcription DP ou une portion de celui-ci suffisante pour former un hétérodimère avec un facteur de transcription E2F, où ledit facteur de transcription DP est incapable de se localiser dans le noyau et (ii) un facteur de transcription E2F ou une portion de celui-ci suffisante pour former un hétérodimère avec le facteur de transcription DP ou une portion de celui-ci et la localisation directe dudit hétérodimère au noyau ; et
b) la détermination du degré de localisation nucléaire dudit hétérodimère en présence et en l'absence d'un antagoniste putatif de la progression du cycle cellulaire afin de déterminer si ledit antagoniste putatif est ou non un antagoniste du cycle cellulaire.

21. Essai selon la revendication 20, où le facteur de transcription DP est DP-1.

22. Essai selon la revendication 20 ou 21, où E2F est E2F-1.
